(19) Europäisches Patentamt
European Patent Office
Office européen des brevets

(11) Veröffentlichungsnummer: **0 094 595**
**B1**

(12) # EUROPÄISCHE PATENTSCHRIFT

(45) Veröffentlichungstag der Patentschrift: **14.01.87**

(21) Anmeldenummer: **83104589.3**

(22) Anmeldetag: **10.05.83**

(51) Int. Cl.⁴: **C 07 D 203/08,** C 07 D 203/02, C 07 D 295/12, C 07 D 409/06, C 07 D 333/04, C 07 C 143/80, A 61 K 31/395, A 61 K 31/38, A 61 K 31/18

(54) **Neue Aziridin- und Phenäthanolaminderivate.**

(30) Priorität: **14.05.82 CH 3013/82**
**16.03.83 CH 1434/83**

(43) Veröffentlichungstag der Anmeldung: **23.11.83 Patentblatt 83/47**

(45) Bekanntmachung des Hinweises auf die Patenterteilung: **14.01.87 Patentblatt 87/3**

(84) Benannte Vertragsstaaten: **AT BE CH DE FR GB IT LI LU NL SE**

(56) Entgegenhaltungen:
**EP-A-0 006 735**
**DD-A-108 532**
**DE-A-2 145 894**
**DE-A-2 843 016**

**CHEMISCHE BERICHTE, 107. Jahrgang, 1974, Weinheim, R. APPEL, R. KLEINSTÜCK, "Eine neue Aziridin-Synthese", Seiten 5-12**

(73) Patentinhaber: **F. HOFFMANN- LA ROCHE & CO. Aktiengesellschaft, CH- 4002 Basel (CH)**

(72) Erfinder: **Alig, Leo, Dr., Liebrütistrasse 32, CH- 4303 Kaiseraugst (CH)**
Erfinder: **Müller, Marcel, Dr., Quellenweg 10, CH- 4402 Frenkendorf (CH)**

(74) Vertreter: **Lederer, Franz, Dr., Vanderwerth, Lederer & Riederer Patentanwälte Lucile- Grahn- Strasse 22, D-8000 München 80 (DE)**

EP 0 094 595 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft neue Aziridinderivate, Verfahren zu ihrer Herstellung, neue Zwischenprodukte dazu und pharmazeutische Präparate auf der Basis der neuen Verbindungen.

Die erfindungsgemässen Aziridinderivate sind Verbindungen der Formel

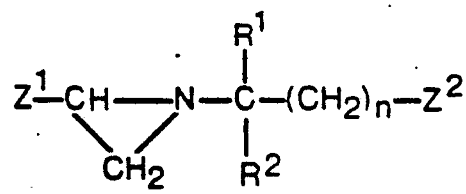

I

worin $Z^1$ ein Rest der Formel

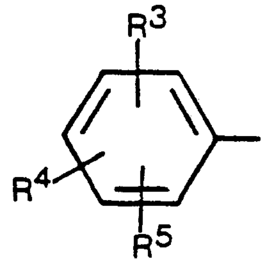

$Z^2$ ein Rest der Formel

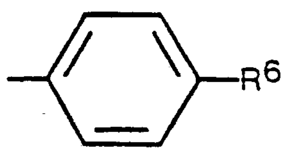

oder

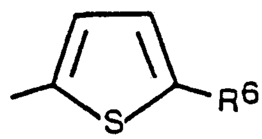

$R^1$ und $R^2$ Wasserstoff oder nieder-Alkyl; $R^3$ Wasserstoff, Halogen, Hydroxy, nieder-Alkyl, nieder-Alkoxy, Amino, nieder-Alkoxybenzylamino, Nitro oder Trifluormethyl, $R^4$ und $R^5$ Wasserstoff oder Halogen, $R^6$ nieder-Alkyl, nieder-Alkanoyl, Cyano, Hydroxy, oder einen Rest $-C(R^7)=C(R^8)COOR^9$, $-CH_2R^{10}$ oder $-C(O)R^{10}$; $R^7$, $R^8$ und $R^9$ Wasserstoff oder nieder-Alkyl; $R^{10}$ Amino, Mono- oder Di-nieder-alkylamino, den Aetherrest eines niederaliphatischen, Alkohols wobei "nieder" nich auf Reste mit bis zu 6 C-Atomen bezieht und n eine ganze Zahl von 1-4 ist, sowie physiologisch verträgliche Salze davon.

Der hier verwendete Ausdruck "nieder" bezeichnet Reste mit 1-6 Kohlenstoffatomen, wobei Reste mit 1-4 Kohlenstoffatomen bevorzugt sind. Alkyl- und Alkoxygruppen können geradkettig oder verzweigt sein. Beispiele sind Methyl, Aethyl, Propyl, Isopropyl, n-Butyl und Isobutyl bzw. Methoxy, Aethoxy, Propoxy, Isopropoxy, Butoxy und Isobutoxy. Acylreste leiten sich von aliphatischen, araliphatischen oder aromatischen Carbönsäuren ab, z.B. nieder-Alkancarbonsäuren, wie Ameisensäure, Essigsäure, Propionsäure und Buttersäure; oder Phenyl-nieder-alkancarbonsäuren, wie Phenylessigsäure; oder Benzoesäure. Beispiele von Alkoholresten $R^{10}$ sind nieder-Alkoxygruppen; Cyclohexyloxy und Cyclopentyloxy; sowie Benzyloxy und substituierte Benzyloxygruppen,wie p-Methoxybenzyloxy. Halogen ist vorzugsweise Chlor oder Brom.

Die Verbindungen der Formel I bilden mit Säuren Salze, die eben alls Gegenstand der Erfindung sind. Beispiele solcher Salze sind Salze mit physiologisch verträglichen Mineralsäuren, wie Salzsäure, Bromwasserstoffsäure, Schwefelsäure, Phosphorsäure; oder mit organischen Säuren, wie Methansulfonsäure, Essigsäure, Propionsäure, Zitronensäure, Bernsteinsäure, Apfelsäure, Fumarsäure, Phenylessigsäure oder Salicylsäure. Carbonsäuren der Formel I können als Salze vorliegen. Beispiele für solche Salze sind Alkalimetall-, Erdalkalimetall-, Ammonium- und Alkylammoniumsalze, wie Na-, K-, Ca-, Trimethylammonium- und Aethanolammoniumsalze.

Die Verbindungen der Formel I enthalten ein oder mehrere asymmetrische Kohlenstoffatome und können somit als optisch aktive Enantiomere, als Diastereomere oder als Racemate vorliegen.

Die Verbindungen der Formel I uhd ihre Salze können erfindungsgemäss dadurch erhalten werden, dass man eine Verbindung der Formel

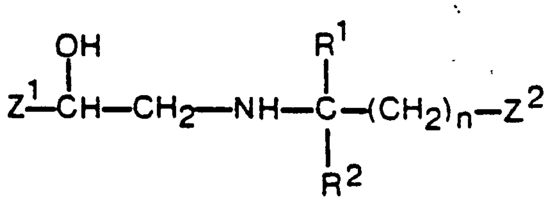

II

worin $Z^1$, $Z^2$, $R^1$, $R^2$ und n die oben angegebene Bedeutung haben,

dehydratisiert gewünschtenfalls einen im Rest $R^2$ enthaltenen reaktionsfähigen Substituenten funktionell abwandelt und/oder das Reaktionsprodukt in ein physiologisch verträgliches Salz überführt.

Die Dehydratisierung einer Verbindung der Formel kann mittels Triphenylphosphin/Tetrachlorkohlenstoff als Dehydratisierungsreagenz (Chem. Ber. 107, 1974, 5) vorgenommen werden. Diese Reaktion wird zweckmässig in einem inerten organischen Lösungsmittel, wie Acetonitril, in Gegenwart einer Base, z.B. einem Amin, wie Triathylamin, durchgeführt. Die Reaktionstemperatur ist nicht kritisch, zweckmässig arbeitet man bei Temperaturen zwischen Raumtemperatur und dem Siedepunkt des Reaktionsgemisches, wobei Raumtemperatur oder leicht erhöhte Temperatur, z.B. 50° C, bevorzugt ist.

Die Dehydratisierung einer Verbindung der Formel II kann auch zweistufig nach den Methoden von Gabriel bzw. Wenker erfolgen, indem man die Hydroxygruppe durch ein Halogenatom ersetzt, z.B. durch Behandlung mit einem Halogenierungsmittel wie Thionylchlorid oder Phosphortrichlorid; oder in eine abspaltbare Gruppe, z.B. in einen Schwefelsäureester, überführt und worauf man das so erhaltene β-Haloamin bzw. das β-Aminohydrogensulfat mit Alkali, z.B. Alkalimetallhydroxiden, wie Natriumhydroxid, behandelt.

Als funktionelle Abwandlung eines im Rest $Z^2$ der Verbindungen der Formel I enthaltenen Substituenten kommen z.B. die Veresterung einer Carboxygruppe, die Verseifung einer Estergruppe, die z.B. als Substituent $-C(O)R^{10}$ anwesend sein kann, und die Umwandlung einer Carbamoylgruppe $R^6$ in eine Nitril- oder Aminomethylgruppe in Betracht.

Die Veresterung kann in an sich bekannter Weise, z.B. mittels Alkylhalogeniden, wie Methyljodid, und einer Base, vorgenommen werden.

Die Verseifung einer Estergruppe wird zweckmässig unter alkalischen Bedingungen, z.B. mittels wässrigalkoholischem Alkalihydroxid, z.B. wässrig-methanolischem Kaliumhydroxid, vorgenommen.

Eine Carbamoylgruppe $R^6$ kann mittels Triphenylphosphin/ Tetrachlorkohlenstoff in Gegenwart einer Base, z.B. einem Amin, wie Triäthylamin, in einem Lösungsmittel, wie Acetonitril, zur Cyangruppe dehydratisiert werden.

Eine Carbamoylgruppe R kann z.B. mittels Lithiumaluminiumhydrid in einem Lösungsmittel, wie Tetrahydrofuran, zur-Aminomethylgruppe reduziert werden.

Gewisse Ausgangsstoffe der Formel II sind bekannt, z.B. aus den europäischen Patentanmeldungen 21636 A1 und 6735 A1.

Die Verbindungen der Formel

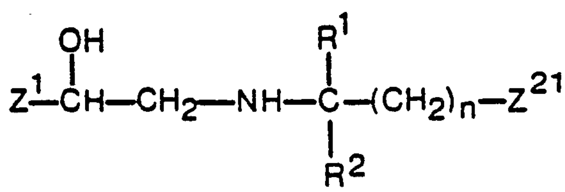

II—I

worin $Z^{21}$ einen Rest

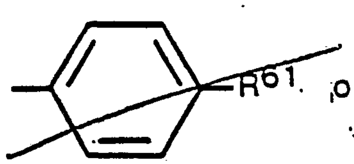 oder 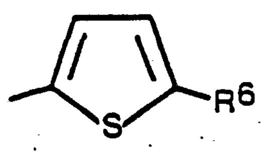

dargstellt, und $Z^1$, $R^1$, $R^2$, $R^6$, und $R^{10}$ die früher angegebene Bedeutung haben,

und deren physiologisch verträglichen Salze sind neu und ebenfalls Gegenstand der Erfindung. Weiterhin betrifft die vorliegende Erfindung pharmazeutische Präparate auf der Basis der Verbindungen der Formel II-1 sowie ein Verfahren zur Herstellung dieser Verbindungen.

Die Verbindungen der Formel II-1 können dadurch erhalten werden dass man
a) eine Verbindung der Formel

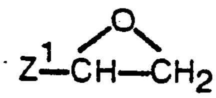

III

mit einer Verbindung der Formel

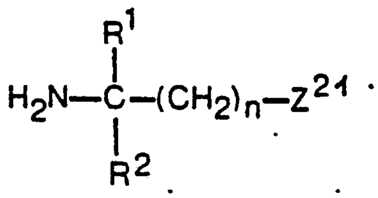

IV

umsetzt; oder
b) eine Verbindung einer der Formeln

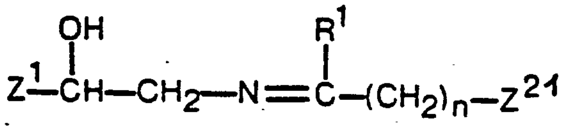

V

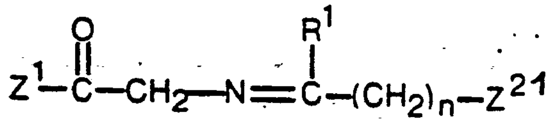

VI

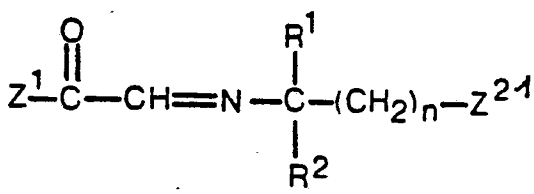

VII

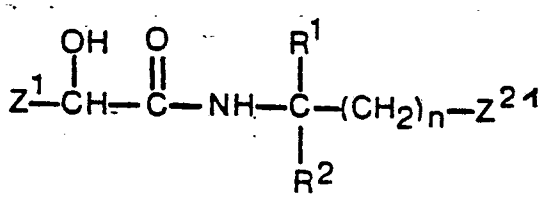

VIII

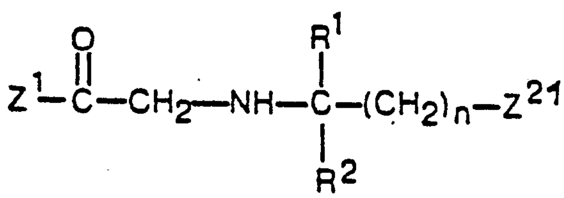

IX

reduziert,
wobei in den vorstehenden Formeln die Reste $Z^1$, $Z^{21}$, $R^2$, und n die früher angegebene Bedeutung haben.

Die Umsetzung einer Verbindung der Formel III mit einer Verbindung der Formel IV kann in einem inerten organischen Lösungsmittel, zweckmässig einem protischen Lösungsmittel wie einem niederen Alkanol, z.B. Aethanol, nommen werden. Die Reaktionstemperatur ist nicht kritisch sie kann zwischen Raumtemperatur und der Rückflusstemperatur des Reaktionsgemisches liegen.

Die Reduktion einer Verbindung der Formel V kann durch katalytische Hydrierung, z.B. in Gegenwart von Edelmetallkatalysatoren, wie Pd- oder Pt-Katalysatoren oder durch Behandlung mit einem komplexen Metallhydrid, wie $NaBH_4$ vorgenommen werden. Hierbei können die für derartige Reduktionen üblichen Reaktionsbedingungen angewandt werden. Die katalytische Hydrierung wird zweckmässig in einem inerten organischen Lösungsmittel wie einem niederen Alkanol, z.B. Aethanol, bei Raumtemperatur oder schwach erhöhter Temperatur, z.B. bei 20-80°C durchgeführt. Die Reduktion mit einem komplexen Metallhydrid wird zweckmässig in einem niederen Alkanol, z.B. Methanol, bei Temperaturen von 20-30°C durchgeführt.

Die Verbindungen der Formeln VI, VII, VIII und IX können mit einem komplexen Metallhydrid in Analogie zu den Verbindungen der Formel V reduziert werden. Ein geeignetes komplexes Metallhydrid für die Reduktion der Verbindungen VI und VII ist $NaBH_4$. Die Verbindungen VIII werden zweckmässig mit $LiAlH_4$ reduziert.

Bevorzugte Verbindungen der Formeln I und II-1 sind diejenigen, worin $R^1$ Wasserstoff und $R^2$ Wasserstoff oder Methyl, insbesondere Methyl; und/oder worin $R^3$ Wasserstoff, nieder-Alkyl, nieder-Alkoxy, Halogen oder Trifluormethyl; $R^4$ Wasserstoff oderHalogen und $R^5$ Wasserstoff sind. Besonders bevorzugt sind solche Verbindungen, worin R Wasserstoff, Chlor oder Trifluormethyl, insbesondere in meta-Stellung, und $R^4$ und $R^5$ Wasserstoff sind.

Bevorzugte Verbindungen der Formel I sind diejenigen, worin $R^6$ eine Gruppe $-CH_2R^{10}$ oder $-C(O)R^{10}$ ist, wobei $C(O)R^{10}$, insbesondere Carbamoyl, besonders bevorzugt ist. Beispiele von solchen Verbindungen sind das p-((S)-3-[(S)-2-Phenyl-1-aziridinyl]-butyl]benzamid und die physiologisch verträglichen Salze davon.

Bevorzugte Verbindungen der Formel II-1 sind diejenigen worin $R^6$ eine Gruppe $-CH_2R^{10}$, $-C(O)R^{10}$, oder $-C(R^7)=C(R^8)COOR^9$ ist Besonders bevorzugt sind solche Verbindungen, worin $R^6$ eine Gruppe $-C(O)R^{10}$ oder $-C(R^7)=C(R^8)COOR^9$ ist, worin $R^7$ und $R^9$ nieder-Alkyl und R Wasserstoff sind, insbesondere Carbamoyl oder 2-Aethoxycarbonyl-1-methylvinyl.

Die Verbindungen der Formel I und II-1 und ihre Salze können als Wirkstoffe in pharmazeutischen Präparaten zur Behandlung der Fettsucht und/oder des Diabetes mellitus, insbesondere des obesen Erwachsenendiabetikers verwendet werden. Im Tierexperiment wurde auf Verabreichung von Verbindungen der Formel I und II-1 ein gesteigerter Katabolismus, vor allem der Fette, beobachtet. Weiterhin wurde beobachtet, dass die Verbindungen der Formel I und II-1 die Bildung von braunem Fettgewebe bei Ratten und obes-hyperglykämischen Mäusen stimulieren. Bekanntlich wird Defekten des braunen Fettgewebes eine wesentliche Rolle bei der Entstehung der Fettsucht zugeschrieben. An obes-hyperglykämischen Mäusen haben die Verbindungen der Formeln I und II-1 einen ausgeprägten antidiabetischen Effekt, indem sie hypoglykämisch wirken und die Glukosurie vermindern. Die Verbindungen der Formel I und II-1 zeigen nur eine geringe Wirkung auf Herztätigkeit und Kreislauf. Die Dosierung kann in Abhängigkeit von der Wirkungsstärke der einzelnen Verbindungen und den individuellen Bedürfnissen des Patienten 0,5-1000 mg, vorzugsweise 2-200 mg pro Tag für einen Erwachsenen betragen, wobei die Dosis als Einzeldosis oder in mehreren Dosen über den Tag verteilt verabreicht werden kann.

Die pharmazeutischen Präparate enthalten den Wirkstoff zusammen mit einem verträglichen pharmazeutischen, organischen oder anorganischen Trägermaterial, wie Wasser, Gelatine, Gummi arabicum, Lactose, Stärke, Magnesiumstearat, Talk, pflanzliche Oele, Polyalkylenglykole oder Vaseline. Die pharmazeutischen Präparate werden vorzugsweise oral, z.B. in Form von Tabletten, Kapseln, Pillen, Pulver, Granulaten, Lösungen, Sirupen, Suspensionen oder Elixiren verabreicht. Die Verabreichung kann aber auch parenteral, z.B. in Form von sterilen Lösungen, Suspensionen oder Emulsionen, erfolgen. Die pharmazeutischen Präparate können sterilisiert sein und/oder Bestandteile enthalten, wie Konservierungsmittel, Stabilisatoren, Netzmittel, Emulgatoren, Salze, um den osmotischen Druck zu variieren, und Puffersubstanzen.

Die Aktivität der neuen Verbindungen der Formeln 1 und II-1 wird aus den nachstehenden Versuchsresultaten deutlich:

1) Wirkung auf den Sauerstoffverbrauch

Männliche Albinoratten im Gewicht von 160-180 g wurden nach 24 Stunden Fasten in Stoffwechselkäfige gesetzt. Die Käfige wurden mit konstant 5 Liter Raumluft/Stunde, die bei einem Taupunkt von 11°C äquilibriert wurde, belüftet. Von der Abluft wurden nach erneuter Aequilibrierung während Perioden von jeweils 14 Minuten Proben gesammelt und der Sauerstoff- und $CO_2$-Gehalt analysiert. Nach einer Anpassungszeit von 4 Stunden erhielten die in Gruppen zu 6 aufgeteilten Tiere entweder Placebo (5% Gummi arabicum) oder die Testsubstanz (suspendiert in 5% Gummi arabicum) per os. Danach wurden 12 Stunden lang die Bestimmungen durchgeführt. In Tabelle I ist der Prozentsatz des gemittelten Sauerstoffverbrauchs nach Medikation während der ersten 3 Stunden und der gesamten Versuchsdauer (12 Stunden) vom Sauerstoffverbrauch der Anpassungsperiode angegeben, wobei entsprechende Korrekturen für Aenderungen in der Placebo-Gruppe berücksichtigt wurden.

**Tabelle I**

| Verbindung hergestellt in Beispiel No. | Dosis µM/kg | $O_2$-Verbrauch % vom Wert der Vorperiode 1.-3. Stunde | 1.-12. Stunde |
|---|---|---|---|
| 1 | 30 | 151 | 112 |
| 2 | 10 | 132 | 109 |
| 4 | 10 | 142 | 120 |
| 6 | 100 | 117 | 111 |
| 8 | 1 | 150 | 114 |
| 9 | 100 | 145 | 119 |
| 10 | 30 | 142 | 114 |
| 11 | 10 | 160 | 118 |
| 12 | 30 | 123 | 108 |
| 14 | 300 | 142 | 137 |
| 15 | 100 | 146 | 127 |
| 16 | 100 | 131 | 106 |
| 17 | 300 | 169 | 137 |
| 18 | 300 | 118 | 122 |
| 21 | 30 | 162 | 124 |
| 22 | 100 | 125 | 122 |
| 23 | 10 | 137 | 111 |
| 24 | 100 | 121 | 110 |
| 25 | 100 | 116 | 109 |
| ~~27~~ | ~~10~~ | ~~127~~ | ~~108~~ |
| ~~28~~ | ~~30~~ | ~~152~~ | ~~122~~ |
| ~~29~~ | ~~3~~ | ~~133~~ | ~~114~~ |
| 30 | 3 | 120 | 110 |
| 31 | 100 | 170 | 138 |
| 32 | 300 | 160 | 131 |
| 33 | 100 | 155 | 125 |
| 36 | 30 | 158 | 122 |
| 37 | 100 | 172 | 127 |
| 38 | 1 | 154 | 119 |
| 39 | 10 | 134 | 111 |

2. Katabole Wirkung auf die Lipide

Gruppen von 4 männlichen Albinoratten im Gewicht von 320-360 g wurden ohne Zugang zu Futter in Stoffwechselkäfigen gehalten. Sauerstoffverbrauch und $CO_2$-Produktion wurde während 12 Stunden

gemessen. Nach 4 Stunden erhielten die Tiere Placebo (5% Gummi arabicum) oder die Testsubstanz (suspendiert in Gummi arabicum) per os. In der Tabelle II ist die gemittelte Abnahme des respiratorischen Quotienten ($CO_2/O_2$) während 8 Stunden nach Verabreichung der Testsubstanz im Vergleich zu den letzten 3 Stunden vor Verabreichung der Testsubstanz angegeben. In den Placebo-Gruppen aufgetretene Aenderungen wurden bei der Berechnung berücksichtigt.

**Tabelle II**

| Verbindung hergestellt in Beispiel No. | Dosis µM/kg | Aenderung des respiratorischen Quotienten |
|---|---|---|
| 5 | 30 | -0,020 |

3. Wirkung auf Harn- und Blutglucose und die Bildung von braunem Fettgewebe

Weibliche hyperglykämische Fettmäuse wurden an eine auf 3 g/Tag/Tier begrenzte Futtermenge angepasst. Die Testverbindungen (suspendiert in 5% Gummi arabicum) oder Placebo (5% Gummi arabicum) wurden während 15 Tagen zweimal täglich oral verabreicht. Harn wurde während 6 Tagen wöchentlich gesammelt und Harnglucose bestimmt. Blutglucose und das Gewicht des interskapulären braunen Fettgewebes wurde am Versuchsende bestimmt.

Die Versuchsresultate sind in Tabelle III als Prozentsätze der Kontrollwerte angegeben.

**Tabelle III**

| Verbindung hergestellt in Beisp. No. | Dosis µM/kg pro Tag | Harnglucose 1.Woche | 2.Woche | Blut-glucose | braunes Fettge-webe |
|---|---|---|---|---|---|
| 5 | 180 | 48% | 33% | 53% | 159% |

**Beispiel 1**

Eine Mischung von 1,70 g rac-p-[3-(ß-Hydroxyphen-äthyl)amino]propyl]benzamid, 0,8 ml Triäthylamin, 0,6 ml Tetrachlorkohlenstoff, 1,65 g Triphenylphosphin und 12 ml Acetonitril wurde 2 1/2 Stunden unter Rühren auf 50° erwärmt. Zur Aufarbeitung wurde auf Eiswasser gegossen, mit Methylenchlorid extrahiert, der Methylenchloridextrakt mit Wasser gewaschen, mit $Na_2SO_4$ getrocknet und im Vakuum eingedampft. Der Rückstand wurde auf 150 g Silicagel chromatographiert. Mit einem Gemisch von Chloroform, n-Propanol und 25% wässrigem Ammoniak (800:40:2) konnte 1,0 g reines p-[3-(2-Phenyl-1-aziridinyl)propyl]benzamid vom Schmelzpunkt 89-90° ($CH_2Cl_2$-Aether) isoliert werden. $\varepsilon_{227} = 14850$; $\varepsilon_{236} = 14300$.

**Beispiel 2**

Gemäss Beispiel 1 wurde aus p-[3-[[(R)-β-Hydroxy-phenäthyl]amino]propyl]benzamid das p-[3-[(S)-2-Propyl-1-aziridinyl]propyl]benzamid vom Schmelzpunkt 101-106° erhalten. $[\alpha]_D^{28} = +89°$ (c = 0,5 in Methanol); $\varepsilon_{224} = 15900$.

**Beispiel 3**

Gemäss Beispiel 1 wurde aus p-[3-[[(S)-β-Hydroxy-phenäthyl]amino]propyl]benzamid das p-[3-[(R)-2-Phenyl-1-aziridinyl]propyl]benzamid erhalten. Smp. 106-108°; $[\alpha]_D^{20} = -95,5°$ (c = 1,0 in Methanol); $\varepsilon_{222} = 16000$, $\varepsilon_{236} = 14700$.

**Beispiel 4**

Gemäss Beispiel 1 wurde aus p-[(S)-3-[[(R)-β-Hydroxy-phenäthyl]amino]butyl]benzamid das p-[S-3-[(S)-2-Phenyl-1-aziridinyl]butyl]benzamid erhalten. Smp. 129-130°; $[\alpha]_D^{20}$ = +125,5° (c = 1,0 in Methanol); $\varepsilon_{222}$ = 15570, $\varepsilon_{236}$ = 14150 (Schulter).

**Beispiel 5**

Zu einer Lösung von 3,1 g p-[(S)-3-[(S)-2-Phenyl-1-aziridinyl]butyl]benzamid in 150 ml Aethylacetat tropfte man bei 5° unter Rühren eine Lösung von 0,87 g Chlorwasserstoff in 20 ml Aethylacetat. Der ausgefallene Niederschlag wurde abgenutscht und aus Methanol-Aether umkristallisiert. Man erhielt reines p-[(S)-3-[(S)-2-Phenyl-1-aziridinyl]-butyl]benzamid-dihydrochlorid, Smp. 186-188°.

**Beispiel 6**

Gemäss Beispiel 1 wurde aus p-[(S)-3-[[(S)-B-Hydroxy-phenäthyl]amino]butyl]benzamid das p-[(S)-3-[(R)-2-Phenyl-1-aziridinyl]butyl]benzamid erhalten. Smp. 145-146°. $[\alpha]_D^{20}$ = -64° (c = 0,5 in $CH_3OH$). $\varepsilon_{226}$ = 16220.

**Beispiel 7**

Gemäss Beispiel 1 wurde aus p-[(S)-3-[[(R)-β-Hydroxy-phenäthyl]amino]butyl]phenol das p-[(S)-3-[(S)-2-Phenyl-1-aziridinyl]butyl]phenol erhalten. Smp. 135-136° (Aether). $[\alpha]_D^{20}$ = +131° (c = 0,6 in Methanol). $\varepsilon_{220}$ = 14880.

**Beispiel 8**

Gemäss Beispiel 1 wurde aus p-[(R)-3-[[(R)-β-Hydroxy-phenäthyl]amino]butyl]benzamid das p-[(R)-3-[(S)-2-Phenyl-1-aziridinyl]butyl]benzamid erhalten. Smp. 145-146° (Aceton-Hexan). $[\alpha]_D^{20}$ = +65° (c = 1,0 in Methanol). $\varepsilon_{226}$ = 6540.

**Beispiel 9**

Gemäss Beispiel 1 wurde aus p-[3-[(β-Hydroxy-m-methoxy-phenäthyl)amino]propyl]benzamid das p-[3-[2-(m-Methoxy-phenyl)-1-aziridinyl]propyl]benzamid erhalten. Smp. 130-132° (aus Aceton-Hexan); $\varepsilon_{226}$ = 18750; $\varepsilon_{274}$ = 2800; $\varepsilon_{280}$ = 2240.

**Beispiel 10**

1,73 g Triphenylphosphin, 1,83 g (R,S)-5-[3-[(β-Hydroxyphenäthyl)amino]propyl]-2-thiophencarbonsäureamid 0,84 ml Triäthylamin, 0,58 ml Tetrachlorkohlenstoff und 12 ml Acetonitril wurden 18 Stunden bei 25° gerührt. Das Reaktionsgemisch wurde im Vakuum eingedampft. Der Rückstand wurde in Methylenchlorid gelöst und dreimal mit eiskalter 2N Salzsäure extrahiert. Die sauren Lösungen wurden mit Natronlauge basisch gestellt und dreimal mit Methylenchlorid extrahiert. Die Methylenchloridlösungen wurden mit Wasser neutralgewaschen, getrocknet und im Vakuum eingedampft. Chromatographie des Rückstandes an Kieselgel mit Aether-Aceton 9:1 gab 800 mg (R,S)-5-[3-(2-Phenyl-1-aziri-dinyl)propyl]-2-thiophencarbonsäureamid, Smp. 117-119° (aus Acetonitril), UV: $\varepsilon_{217}$ = 12290, $\varepsilon_{256}$ = 8470, $\varepsilon_{275}$ = 10620.

**Beispiel 11**

Gemäss Beispiel 10 wurde aus (R)-5-[3-[(β-Hydroxy-phenäthyl)amino]propyl]-2-thiophencarbonsäureamid das (S)-5-[3-(2-Phenyl-1-aziridinyl)propyl]-2-thiophencarbon-säureamid erhalten. Smp. 95-98°; $[\alpha]_D^{20}$ = +96°

(c = 1,0 in Methanol); $\varepsilon_{218}$ = 12100, $\varepsilon_{256}$ = 8830, $\varepsilon_{275}$ = 11200.

**Beispiel 12**

Eine Mischung von 2,98 g p-[3-[[(R)-β-Hydroxyphen-äthyl]amino]propyl]benzamid, 3,0 ml Triäthylamin, 2,2 ml Tetrachlorkohlenstoff, 5,5 g Triphenylphosphin und 100 ml Acetonitril wurde 1 Stunde zum Rückfluss erhitzt. Zur Aufarbeitung wurde das Reaktionsgemisch auf Eiswasser gegossen, mit Aethylacetat extrahiert, der Extrakt mit Wasser gewaschen, mit $Na_2SO_4$ getrocknet und im Vakuum eingedampft. Der Rückstand wurde auf 400 g Silicagel chromatographiert. Mit Hexan-Aceton (9:1) konnten 1,8 g reines p-[3-[(S)-2-Phenyl-1-aziridinyl]prooyl]phenylcyanid als farbloses Oel eluiert werden. $[\alpha]_D^{20}$ = +92,4° (c = 1,0 in Methanol), $\varepsilon_{231}$ = 19600.

**Beispiel 13**

Gemäss Beispiel 1 wurde aus p-[(S)-3-[[(R)-β-Hydroxy-phenäthyl]amino]butyl]benzamid das p-[(S)-3-[(S)-2-Phenyl-1-aziridinyl]butyl]benzamid erhalten.

**Beispiel 14**

Gemäss Beispiel 1 wurde aus p-[3-[(β-Hydroxy-m-trifluormethyl-phenäthyl)amino]propyl]benzamid das p-[3-[2-(m-Trifluormethylphenyl)-1-aziridinyl]propyl]benzamid erhalten. Smp. 135-138° (aus Aceton-Hexan).

**Beispiel 15**

Gemäss Beispiel 1 wurde aus p-[3-[(ß-Hydroxy-m-chlorphenäthyl)amino]propyl]benzamid das p-[3-[2-(m-chlorphenyl)-1-aziridinyl]propyl]benzamid erhalten, Smp. 124-125° (aus Aceton-Hexan).

**Beispiel 16**

Gemäss Beispiel 1 wurde aus rac-p-[3-[(β-Hydroxy-phenäthyl)amino]propyl]benzoesäure-methylester der p-[3-(2-Phenyl-1-aziridinyl)propyl]benzoesäure-methylester erhalten. Oel; $\varepsilon_{214}$ = 11040; $\varepsilon_{239}$ = 17330; $\varepsilon_{270}$ = 1230.

**Beispiel 17**

Gemäss Beispiel 1 wurde aus p-[(R)-3-[[(S)-β-Hydroxy-phenäthyl]amino]butyl]benzamid das p-[(R)-3[(R)-2-Phenyl-1-aziridinyl]butyl]benzamid erhalten. Smp. 122-123° (aus Aceton-Hexan). $\varepsilon_{222}$ = 15630; $\varepsilon_{236}$ = 14400.

**Beispiel 18**

Gemäss Beispiel 1 wurde aus p-[3-[(β-Hydroxy-3,5-dichlorphenäthyl)amino]propyl]benzamid das p-[3-[2-(3',5'-Dichlorphenyl)-1-aziridinyl]propyl]benzamid erhalten; Smp. 155-156° (aus Aceton-Hexan).

**Beispiel 19**

Gemäss Beispiel 1 wurden aus p-[(S)-2-[(R,S)-β-Hydroxy-m-(trifluormethyl)phenäthyl]amino]propyl]-β-methyl-zimtsäure-methylester der β-Methyl-p-[(S)-2-[(R)-2-(α,α,α-trifluor-m-tolyl)-1-aziridinyl]propyl]zimtsäuremethylester als gelbes Oel, $[\alpha]_D^{20}$ = -27° (c = 0,3 in Methanol), $\varepsilon_{274}$ = 20600, und der β-Methyl-p-[(S)-2-[(S)-2-(α,α,α-trifluor-m-tolyl)-1-aziridinyl]propyl]zimtsäuremethylester als gelbes Oel, $[\alpha]_D^{20}$ = +161° (c = 0,5 in Methanol), $\varepsilon_{275}$ = 19100, erhalten.

**Beispiel 20**

Gemäss Beispiel 1 wurden aus p-[(R)-2-[[(R,S)-β-Hydroxy-m-(trifluormethyl)phenäthyl]amino]propyl]-β-methyl-zimtsäure-methylester der β-Methyl-p-[(R)-2-[(S)-2(α,α,α-trifluor-m-tolyl)-1-aziridinyl]propyl]zimtsäuremethylester als gelbes Oel, $[\alpha]_D^{20}$ = +26,4° (c = 0,5 in Methanol), $\varepsilon_{274}$ = 20700, und der β-Methyl-p-[(R)-2-[(R)-2(α,α,α-trifluor-m-tolyl)-1-aziridinyl]propyl]zimtsäuremethylester als gelbes Oel, $[\alpha]_D^{20}$ = -161,6° (c = 0,6 in Methanol), $\varepsilon_{274}$ = 18950, erhalten.

**Beispiel 21**

Gemäss Beispiel 1 wurde aus p-[(R,S)-3-[[(R)-β-Hydroxy-phenäthyl]amino]butyl]-N,N-dimethylbenzamid das p-[(R,S)-3-[(S)-2-Phenyl-1-aziridinyl]butyl]-N,N-dimeth-ylbenzamid erhalten; amorph, $\varepsilon_{218}$ = 18700 (1:3 Diastereomeren-Gemisch).

**Beispiel 22**

Eine Mischung von 2,8 g p-[3-[(S)-2-Phenyl-1-aziri-dinyl]propyl]benzamid und 1,5 g $LiAlH_4$ in 400 ml absolutem Tetrahydrofuran wurde 6 Stunden bei Raumtemperatur gerührt. Dann wurde tropfenweise mit 3 ml gesättigter $Na_2SO_4$-Lösung versetzt und anschliessend der weisse Niederschlag abfiltriert und mit Methylenchlorid nachgewaschen. Das Filtrat wurde im Vakuum eingedampft und der Rückstand auf 200 g Kieselgel chromatographiert. Mit Chloroform-n-Propanol-ges. $NH_3$-Lösung (800:50:3) konnten 1,95 g reines p-[3-[(S)-2-Phenyl-1-aziridinyl]propyl]benzylamin als farblose Flüssigkeit eluiert werden.

**Beispiel 23**

Gemäss Beispiel 1 wurde aus p-[(S)-3-[[(R)-β-Hydroxy-phenäthyl]amino]butyl]N-methylbenzamid das p-[(S)-3[(S)-2-Phenyl-1-aziridinyl]butyl]-N-methylbenzamid erhalten. Smp. 113-114°. $[\alpha]_D^{20}$ = 120 7° (c = 0,4 in $CH_3OH$).

**Beispiel 24**

Gemäss Beispiel 1 wurde aus p-[3-[[(R)-β-Hydroxy-phenäthyl]amino]propyl]toluol das p-[3[(S)-2-Phenyl-1-aziridinyl]propyl]toluol als farblose Flüssigkeit erhalten. $[\alpha]_D^{20}$ = +101,5° (c = 1,0 in Methanol); $\varepsilon_{217}$ = 16030.

**Beispiel 25**

Gemäss Beispiel 1 wurde aus p-[3-[[(R)-β-Hydroxy-phenäthyl]amino]propyl]benzol das p-[3-[(S)-2-Phenyl-1-aziridinyl]propyl]benzol als farblose Flüssigkeit erhalten. $[\alpha]_D^{20}$ = +100° (c = 1,0 in Methanol); $\varepsilon_{216}$ = 12150.

**Beispiel 26**

10 g 5-(3-Aminopropyl)-2-thiophen-carbonsäureamid und 6,8 ml (R,S)-Phenyläthylenoxid wurden in 136 ml DMSO 18 Stunden auf 95° erwärmt. Das Reaktionsgemisch wurde mit Wasser und Methylenchlorid verdünnt, die wässerige Phase wurde zweimal mit Methylenchlorid extrahiert; die Methylenchloridphasen wurden mit Wasser gewaschen, über Natriumsulfat getrocknet und im Vakuum eingedampft. Chromatographie des Rückstandes mit Methanol an Kieselgel gab 7,85 g (R,S)-5-[3-[(β-Hydroxyphenäthyl)amino]propyl]-2-thiophencarbonsäureamid, Smp. 116-117° (aus Methanol-Aether). UV: $\varepsilon_{257}$ = 8830, $\varepsilon_{276}$ = 10990.

**Beispiel 27**

Analog Beispiel 26 wurde aus 5-(3-Aminopropyl)-2-thiophencarbonsäureamid und (R)-Phenyläthylenoxid das (R)-5-[3-[(β-Hydroxyphenäthyl)amino]propyl]-2-thiophencar-bonsäureamid erhalten. Smp. 89-93°, $[\alpha]_{589}$ = -19° (c = 0,1% in Dioxan), UV: $\varepsilon_{256}$ - 8600, $\varepsilon_{276}$ - 10790.

**Beispiel 28**

Analog Beispiel 26 wurde aus 5-(3-Aminopropyl)-2-thiophencarbonsäureamid und (S)-Phenyläthylenoxid das (S)-5-[3-[(ß-Hydroxyphenäthyl)amino]propyl]-2-thiophencar-bonsäureamid erhalten. Smp. 94-96°, $[\alpha]_{589}$ = +19° (c = 0,1% in Dioxan); UV: $\varepsilon_{256}$ = 8490, $\varepsilon_{275}$ = 10780.
Das Ausgangsamid kann wie folgt hergestellt werden:
2-(p-Toluolsulfonyloxy)propylthiophen (J. Org. Chem. 36, 1971, 2236) wurde mit Acetylchlorid und Aluminiumtrichlorid in Methylenchlorid zu 5-Acetyl-2-(p-toluolsul-fonyloxy)propylthiophen umgesetzt. Mit Natriumazid in DMSO wurde daraus 5-(3-Azidopropyl)-2-thienyl-methylketon erhalten. Oxidation mit Hypobromid gab 5-(3-Azidopropyl)-2-thiophen-carbonsäure, Smp. 71-72°.
Umsetzen dieser Säure mit Thionylchlorid und anschliessende Behandlung mit konz. Ammoniak führte zum 5-(3-Azidopropyl)-2-thiophencarbonsäureamid, Smp. 85-87°. Daraus erhält man nach Behandlung mit Triphenylphosphin und Hydrolyse (J. Org. Chem. 40, 1975, 1659) 5-(3-Aminopropyl)-2-thiophen-carbonsäureamid, Smp. 143,5-144° (aus Wasser).

**Beispiel 29**

2,58 g 5-[3-[[(R)-ß-Hydroxyphenäthyl]amino]propyl]-2-thiophencarbonsäureamid wurden in 129 ml Tetrahydrofuran unter Argon portionenweise mit 1,29 g Lithiumaluminiumhydrid versetzt und 3 1/4 Stunden am Rückfluss gekocht.
Dann wurden unter Kühlung vorsichtig 34 ml 2N Natronlauge und anschliessend 400 ml Wasser zugegeben und dreimal mit Chloroform extrahiert. Die Chloroformlösungen wurden mit Wasser und verdünnter Kochsalzlösung neutral gewaschen, getrocknet und im Vakuum eingedampft. Chromatographie an Kieselgel mit Methanol und 0,5% konz. Ammoniak und Kristallisation aus Acetonitril-Aether gab 914 mg (R)-α-[[[3-[5-(Aminomethyl)-2-thienyl]propyl]amino]methyl]benzylalkohol, Smp. 64-65°, $[\alpha]_D$ = -18° (in Dioxan, c = 0,1%), UV: $\varepsilon_{240}$ 9080.

**Beispiel 30**

Analog Beispiel 29 wurde aus (R,S)-5-[3-[(β-Hydroxy-phenäthyl)amino]propyl]-2-thiophencarbonsäureamid der (R,S)-α-[[[3-[5-(Aminomethyl)-2-thienyl]propyl]amino]-methyl]benzylalkohol erhalten, Smp. 89-90°, UV: $\varepsilon_{240}$ = 8320.

**Beispiel 31**

9,91 g (R,S)-5-(3-Aminobutyl)-2-thiophencarbonsäure-amid und 5,7 ml (R)-Phenyläthylenoxid wurden in 100 ml DMSO 47 Stunden auf 100° erwärmt. Das Reaktionsgemisch wurde auf Wasser gegossen und dreimal mit Methylenchlorid extrahiert; die Methylenchloridlösungen wurden mit Wasser und verdünnter Kochsalzlösung gewaschen, getrocknet und im Vakuum eingedampft. Chromatographie des Rückstands an Kieselgel mit Aether-MeOH 4:1 gab 7,35 g 5-[(R,S)-3-[[(R)-β-Hydroxyphenäthyl]amino]butyl]-2-thiophencarbonsäureamid, Smp. 124-125° (aus Acetonitril); $[\alpha]_D$ = -24° (in Dioxan, c = 0,1%); UV: $\varepsilon_{256}$ = 8500, $\varepsilon_{275}$ = 10830.
Das Ausgangsmaterial kann wie folgt hergestellt werden:
4-(5-Acetyl-2-thienyl)-2-butanon (Tetrahedron 35, 1979, 329) wurde mit Aethylenglykol, o-Ameisensäuretriäthylester und p-Toluolsulfonsäure in Methylenchlorid selektiv zu Methyl 5-[2-(2-methyl-1,3-dioxolan-2-yl)äthyl]-2-thienyl-keton umgesetzt. Oxidation mit Natriumhypobromit und anschliessende Hydrolyse gab 5-(3-Oxobutyl)-2-thiophencar-bonsäure. Mit $NaBH_4$ wurde daraus 5-(3-Hydroxybutyl)-2-thiophencarbonsäure erhalten, welche in Dimethylacetamid mit Methyljodid und Natriumbicarbonat in den Methylester übergeführt wurde. Behandlung mit p-Toluolsulfochlorid in Pyridin und Reaktion mit Natriumazid in DMSO gab 5-(3-Azidobutyl)-2-thiophencarbonsäuremethylester, aus welchem durch Verseifung die entsprechende Säure erhalten wurde. Mit Thionylchlorid wurde deren Säurechlorid hergestellt, aus dem mit konz. Ammoniak in Aether 5-(3-Azidobutyl)-2-thiophencarbonsäureamid erhalten wurde. Reduktion der

Azidogruppe mit Triphenylphosphin und anschliessende Hydrolyse gab (R,S)-5-(3-Aminobutyl)-2-thiophencarbonsäureamid, Smp. 65-75°, UV: $\varepsilon_{256}$ 7780, $\varepsilon_{275}$ = 9900.

**Beispiel 32**

In zu Beispiel 10 analoger Weise wurden hergestellt:

a) 5-[(RS)-3-[(S)-2-Phenyl-1-aziridinyl]butyl]-2-thio-phencarbonsäureamid, Smp. 76-78°, $[\alpha]_D^{20}$ = +99°, (c = 0,1% in Dioxan), $\varepsilon_{275}$ = 10560,

b) p-[2-[(S)-2-Phenyl-1-aziridinyl]äthyl]benzamid, Smp. 121-122°, $[\alpha]_D^{20}$ = +113° (c = 0,1% in MeOH), $\varepsilon_{225}$ = 15930,

c) 5-[3-(S)-(2-Phenyl-1-aziridinyl)propyl]-2-thiophen-carbonsäuremethylester, $[\alpha]_D^{20}$ = +95° (c = 0,1% in Dioxan), $\varepsilon_{278}$ = 12420, $\varepsilon_{254}$ = 9360.

Der zur Herstellung des Aziridins in Absatz c) verwendete 5-(3-Aminopropyl)-2-thiophencarbonsäuremethylester kann dadurch hergestellt werden, dass man die 5-(3-Azido-propyl)-2-thiophencarbonsäure mit Methyljodid verrestert und den dabei erhaltenen 5-(3-Azidopropyl)-2-thiophencar-bonsäuremethylester katalytisch hydriert.

**Beispiel 33**

In zu Beispiel 26 analoger Weise wurden hergestellt:

a) 5-[3-[[(R)-β-Hydroxyphenäthyl]amino]propyl]-2-thiophen-carbonsäuremethylester, Smp. 81-82°, $[\alpha]_D^{20}$ = -15° (c = 0,1% in Dioxan); $\beta_{254}$ = 9340, $\beta_{277}$ = 12410,

b) (E)-5-[(RS)-3-[[(R)-β-Hydroxyphenäthyl]amino]butyl]-β-methyl-2-thiophenacrylsäureäthylester, Smp. 72°, $[\alpha]_D^{20}$ -19° (c = 0,1% in Dioxan), $\varepsilon_{326}$ = 18690,

c) 5-[2-[[(R)-β-Hydroxyphenäthyl]amino]äthyl]-2-thiophen-carbonsäureamid, Smp. 95-97°; $[\alpha]_D^{20}$ = -27° (c = 0,1% in MeOH); $\varepsilon_{257}$ = 9000, $\varepsilon_{275}$ = 10520,

d) 5-[(RS)-2-[[(R)-β-Hydroxyphenäthyl]amino]propyl]-2-thiophencarbonsäureamid, $[\alpha]_D^{20}$ = -18° (c = 0,1% in Dioxan); $\varepsilon_{257}$ = 8560, $\varepsilon_{275}$ = 10340,

e) (E)-5-[(RS)-3-[[(RS)-β-Hydroxy-m-(trifluormethyl)-phenäthyl]amino]butyl]-β-methyl-2-thiophenacrylsäureäthylester, $\varepsilon_{321}$ = 16730,

f) 5-[(RS)-3-[[(RS)-β-Hydroxy-m-(trifluormethyl)phen-äthyl]amino]butyl]-2-thiophencarbonsäureamid, Smp. 165-166°, $\varepsilon_{275}$ - 11000,

g) (E)-5-[(RS)-2-[[(R)-β-Hydroxyphenäthyl]amino]propyl]-β-methyl-2-thiophencarbonsäureäthylester, $[\alpha]_D^{20}$ = -14° (c = 0,1% in Dioxan), $\varepsilon_{320}$ - 17130,

h) (E)-5-[(RS)-2-[[(RS)-β-Hydroxy-m-(trifluormethyl)-phenäthyl]amino]propyl]-β-methyl-2-thiophenacrylsäureäthylester, Smp. 101-102°, $\varepsilon_{321}$ = 19140,

i) 5-[(RS)-3-[[(R)-β-Hydroxyphenäthyl]amino]butyl]-2-thienylmethylketon, Smp. 66-68°, $[\alpha]_D^{20}$ = -19° (c = 0,1% in Dioxan), $\varepsilon_{264}$ = 8730, $\varepsilon_{294}$ = 11940,

j) 5-[(RS)-2-[[(R)-β-Hydroxyphenäthyl]amino]propyl]-2-thienylmethylketon, $[\alpha]_D^{20}$ = -23° (c = 0,1% in MeOH), $\varepsilon_{264}$ = 8410, $\varepsilon_{294}$ = 11260,

k) 5-[(RS)-3-[[(R)-ß-Hydroxyphenäthyl]amino]butyl]-2-thiophencarbonsäuremethylester, Smp. 102-103°, $[\alpha]_D$ = -27° (c = 0,1% in MeOH), $\varepsilon_{255}$ = 9500, $\varepsilon_{279}$ = 12620.

Der zur Herstellung der Alkohole in den Absätzen b) und e) verwendete (E)-5-[(RS)-3-Aminobutyl]-ß-methyl-2-thio phenacrylsäureäthylester kann wie folgt hergestellt werden:

(RS)-4-(2-Thienyl)-2-butanol (Coll. Czech. Chem. Comm. 41, 1976, 479), Acetylchlorid und Aluminiumchlorid wurden in Methylenchlorid zu (RS)-3-(5-Acetyl-2-thienyl)-1-methyl-propylacetat umgesetzt. Mit NaOH in Methanol wurde dieses zu (RS)-5-(3-Hydroxybutyl)-2-thienylmethylketon verseift. Dieses reagierte in Alkohol in Gegenwart von Natriumalkoholat mit Triäthylphosphonoacetat zu (E)-5-[(RS)-3-Hydroxy-butyl]-ß-methyl-2-thiophenacrylsäureäthylester. Umsetzen mit p-Toluolsulfochlorid und anschliessende Behandlung mit Natriumazid gab (E)-5-[(RS)-3-Azidobutyl]-β-methyl-2-thio-phenacrylsäureäthylester. Daraus wurde durch Reduktion mit Triphenylphosphin und anschliessende Hydrolyse (E)-5-[(RS)-3-Aminobutyl]-ß-methyl-2-thiophenacrylsäureäthylester erhalten, $\varepsilon_{320}$ = 17465.

Das zur Herstellung des Alkohols im Absatz c) verwendete 5-(2-Aminoäthyl)-2-thiophencarbonsäureamid kann wie folgt hergestellt werden.

2-(2-Thienyl)äthyl-p-toluolsulfonat (J.A.C.S. 95, 1973, 1247) Acetylchlorid und Aluminiumchlorid wurden in Methylenchlorid zu 2-[(5-Acetyl-2-thienyl)äthyl]-p-toluol-sulfonat umgesetzt (Smp. 111-112°, aus Aethanol). Dieses wurde mit Natriumazid in DMSO in das 5-(2-Azidoäthyl)-2-thienylmethylketon übergeführt. Oxidation mit Natriumhypobromit lieferte die 5-(2-Azidoäthyl)-2-thiophencarbonsäure vom Smp. 53-55°, die mit Thionylchlorid das entsprechende Säurechlorid ergab, welches mit Ammoniak zum 5-(2-Azido-äthyl)-2-thiophencarbonsäureamid umgesetzt wurde (Smp. 104-105', aus Aethanol). Reaktion mit Triphenylphosphin und Hydrolyse (J. Org. Chem. 40, 1975, 1659) gab 5-(2-Aminoäthyl)-2-thiophencarbonsäureamid, Smp. 134-136°,

aus Acetonitril.

Das zur Herstellung des Alkohols im Absatz d) verwendete 5-[(RS)-2-Aminopropyl]-2-thiophencarbonsäureamid kann wie folgt hergestellt werden:

α-Methyl-2-thiophenäthanol (J.A.C.S. 64, 1942, 477), Acetylchlorid und Aluminiumchlorid wurden in Methylenchlorid zu (RS)-2-(5-Acetyl-2-thienyl)-1-methyläthylacetat umgesetzt. Mit Natronlauge in Methanol wurde dieses zu 5-[(RS)-2-Hydroxypropyl]-2-thienylmethylketon verseift und anschliessend mit p-Toluolsulfochlorid zu (RS)-2-(5-Acetyl-2-thienyl)-1-methyläthyl-p-toluolsulfonat umgesetzt, Smp. 5 101-103°. Mit Natriumazid in DMSO wurde daraus 5-[(RS)-2-Azidopropyl]-2-thienylmethylketon erhalten, welches mit Brom in Natronlauge zur 5-[(RS)-2-Azidopropyl]-2-thiophen-carbonsäure oxidiert wurde. Mit $SOCl_2$ wurde daraus das entsprechende Säurechlorid, und aus diesem mit Ammoniak 5-[(RS)-2-Azidopropyl]-2-thiophencarbonsäureamid hergestellt, Smp. 79-80°C, aus Aether. Behandlung mit Triphenylphosphin und Hydrolyse gab 5-[(RS)-2-Aminopropyl]-2-thio-phencarbonsäureamid, Smp. 91-92° aus Acetonitril.

Der zur Herstellung der Alkohole in den Absätzen g) und h) verwendete (E)-5-[(RS)-2-Aminopropyl]-ß-methyl-2-thiophenacrylsäureäthylester kann wie folgt hergestellt werden:

5-[(RS)-2-Hydroxypropyl]-2-thienylmethylketon und Triäthylphosphonoacetat wurden in Alkohol in Gegenwart von Natriumäthylat zu (E)-5-[(RS)-2-Hydroxypropyl]-β-methyl-2-thiophenacrylsäureäthylester umgesetzt. Mit p-Toluolsulfochlorid wurde daraus der (E)-β-Methyl-5-[(RS)-2-[(p-toluolsulfonyl)oxy]propyl]-2-thiophenacrylsäureäthylester erhalten (Smp. 121°, aus Methylenchlorid-Alkohol). Reaktion mit Natriumazid in DMSO gab den (E)-5-[(RS)-2-Azidopropyl]-β-methyl-2-thiophenacrylsäureäthylester. Reduktion mit Triphenylphosphin und Hydrolyse führte zum (E)-5-[(RS)-2-Aminopropyl]-ß-methyl-2-thiophenacrylsäureäthylester, $\varepsilon_{320}$ = 17970.

Das zur Herstellung des Alkohols im Absatz i) verwendete 2-Acetyl-5-[(RS)-3-aminobutyl]thiophen kann wie folgt hergestellt werden:

(RS)-5-(3-Hydroxybutyl)-2-thienylmethylketon wird mit p-Toluolsulfochlorid zum (RS)-3-(5-Acetyl-2-thienyl)-1-methylpropyl-p-toluolsulfonat, Smp. 61-63° umgesetzt. Mit Natriumazid erhält man daraus Methyl-(RS)-5-(3-azidobutyl)-2-thienylketon, welches katalytisch zu 2-Acetyl-5-[(RS)-3-aminobutyl]thiophen hydriert wird.

Das zur Herstellung des Alkohols im Absatz j) verwendete 2-Acetyl-5-[(RS)-2-aminopropyl]thiophen kann dadurch hergestellt werden, dass man das 5-[(RS)-2-Azido-propyl]-2-thienylmethylketon mit Triphenylphosphin umsetzt und anschliessend mit wässerigem Ammoniak hydrolysiert.

Das zur Herstellung des Alkohols im Absatz k) verwendete 5-[(RS)-3-Aminobutyl]-2-thiophencarbonsäuremethylester kann dadurch hergestellt werden, dass man den 5-(3-Azidobutyl)-2-thiophencarbonsäuremethylester mit Triphenylphosphin in Pyridin umsetzt und mit konz. Ammoniak zum 5-[(RS)-3-Aminobutyl]-2-thiophencarbonsäuremethylester, $\varepsilon_{278}$ = 11280, $\varepsilon_{254}$ = 8760 hydrolysiert.

**Beispiel 34**

Gemäss Beispiel 1 wurde aus p-[3-[[(R)-β-Hydroxy-phenäthyl]amino]propyl]benzoesäuremethylester der p-[3-[(S)-2-Phenyl-1-aziridinyl]propyl]benzoesäuremethyl-ester als Oel erhalten $\varepsilon_{238}$ = 17130 $[\alpha]_D^{20}$ = +85° (c = 0,8 in Methanol).

**Beispiel 35**

Gemäss Beispiel 1 wurde aus p-[3-(S)-[[(RS)-β-Hydroxy-m-(trifluormethyl)phenäthyl]amino]butyl]-β-methylzimtsäuremethylester der

(E)-β-Methyl-p-[(S)-3-[(R)-2-(α,α,α-trifluor-m-tolyl)-1-aziridinyl]butyl]zimtsäuremethylester als farbloses Oel, $[\alpha]_D^{20}$ = -49° (c = 0,5 in Methanol), $\varepsilon_{274}$ = 18250, und der

(E)-β-Methyl-p-[(S)-3-[(S)-2-(α,α,α-trifluor-m-tolyl)-1-aziridinyl]butyl]zimtsäuremethylester als farbloses Oel, $[\alpha]_D^{20}$ = +100° (c = 0,6 in Methanol), $\varepsilon_{274}$ = 19880,

erhalten.

**Beispiel 36**

Gemäss Beispiel 1 wurde aus p-[3-(R)-[[(RS)-β-Hydroxy-m-(trifluormethyl)phenäthyl]amino]butyl]-ß-methylzimtsäuremethylester der

(E)-β-Methyl-p-[(R)-3-[(S)-2-(α,α,α-trifluor-m-tolyl)-1-aziridinyl]butyl]zimtsäuremethylester als farbloses Oel, $[\alpha]_D^{20}$ = +53° (c = 0,7 in Methanol), $\varepsilon_{274}$ = 19840 und der

(E)-β-Methyl-p-[(R)-3-[(R)-2-(α,α,α-trifluor-m-tolyl)-1-aziridinyl]butyl]zimtsäuremethylester als farbloses Oel, $[\alpha]_D^{20}$ = -104° (c = 1,0 in Methanol), $\varepsilon_{274}$ - 19040

erhalten.

**Beispiel 37**

Gemäss Beispiel 1 wurde aus p-[(R)-3-[[(RS)-β-Hydroxy-m-(trifluormethyl)-phenäthyl]amino]butyl]benzamid das p-[(R)-3-[2-(α,α,α-Trifluor-m-tolyl)-1-aziridinyl]butyl]-benzamid in Form eines etwa 1:4-Gemisches der 2-R- und 2-S-Form, Smp. 149-153° (aus Aether), $[\alpha]_D^{20} = +49°$ (c = 0,7 in Methanol), $\varepsilon_{230}$ = 17820 erhalten.

**Beispiel 38**

Es werden in üblicher Weise Tabletten folgender Zusammensetzung hergestellt:
Wirkstoff der Formel I oder II-1, z.B. p-[(S)-3-[(S)-2-Phenyl-1-aziridinyl]-butyl]benzamid-dihydrochlorid 250 mg
Lactose 200 mg
Maisstärke 300 mg
Maisstärkepaste 50 mg
Calciumstearat 5 mg
Dicalciumphosphat 45 mg

**Patentansprüche** für die Vertragsstaaten BE CH DE FR GB IT LI LU NL

SE
1. Verbindungen der Formel

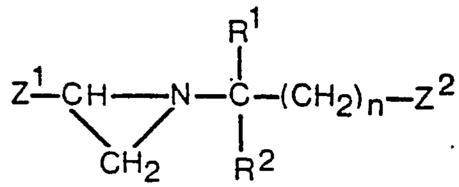

I

worin $Z^1$ ein Rest der Formel

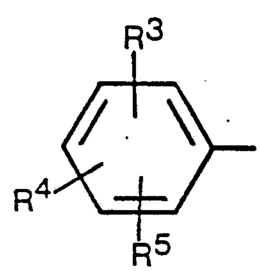

$Z^2$ ein Rest der Formel

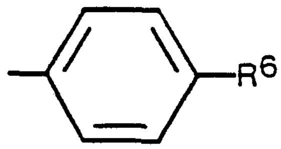

oder

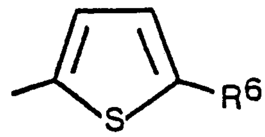

$R^1$ und $R^2$ Wasserstoff oder nieder-Alkyl; $R^3$ Wasserstoff, Halogen, Hydroxy, nieder-Alkyl, nieder-Alkoxy, Amino, nieder-Alkoxybenzylamino, Nitro oder Trifluormethyl; $R^4$ und $R^5$ Wasserstoff oder Halogen; $R^6$ nieder-Alkyl, nieder-Alkanoyl, Cyano, Hydroxy, oder einen Rest $-C(R^7)=C(R^8)COOR^9$, $-CH_2R^{10}$ oder $-C(O)R^{10}$; $R^7$, $R^8$ und $R^9$ Wasserstoff oder nieder-Alkyl; $R^{10}$ Amino, Mono- oder Di-nieder-alkylamino, den Aetherrest eines niederaliphatischen Alkohols wobei "nieder" sich auf Reste mit bis zu 6 C-Atomen bezieht und n eine ganze Zahl von 1-4 is sowie physiologisch verträgliche Salze davon.

2. Verbindungen der Formel I nach Anspruch 1, in denen $R^1$ und $R^2$ Wasserstoff oder nieder-Alkyl, $R^3$ Wasserstoff, Halogen, Hydroxy, nieder-Alkyl, nieder-Alkoxy, Amino, nieder-Alkoxybenzylamino, Nitro oder Trifluormethyl $R^4$ und $R^5$ Wasserstoff oder Halogen, $R^6$ Hydroxy, einen Rest $-C(R^7)=C(R^8)COOR^9$, $-CH_2R^{10}$ oder $-C(O)R^{10}$ sind, und $R^7$, $R^8$, $R^9$ und $R^{10}$ die in Anspruch 1 angegebene Bedeutung haben, sowie physiologisch verträgliche Salze davon.

3. Verbindungen der Formel

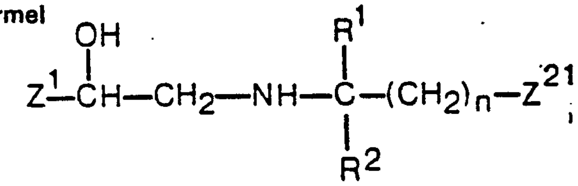

II—I

worin $Z^{21}$ einen Rest

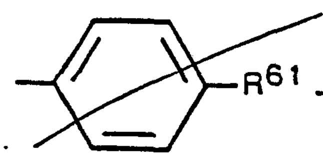

~~oder~~

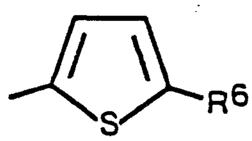

darstellt, und $Z^1$, $R^1$, $R^2$, $R^6$ und $R^{10}$ die in Anspruch 1 angegebene Bedeutung haben, und deren physiologisch verträglichen Salze.

4. Verbindungen gemäss Anspruch 1, 2 oder 3 in denen $R^1$ Wasserstoff und $R^2$ Wasserstoff oder Methyl insbesondere Methyl, sind.

5. Verbindungen gemäss den Ansprüchen 1, 3 oder 4, in denen $R^3$ Wasserstoff, nieder-Alkyl nieder-Alkoxy, Halogen oder Trifluormethyl, $R^4$ Wasserstoff oder Halogen und $R^5$ Wasserstoff sind.

6. Verbindungen gemäss einem der Ansprüche 1-5, in denen $R^3$ Wasserstoff, Chlor oder Trifluormethyl, insbesondere in meta-Stellung, und $R^4$ und $R^5$ Wasserstoff sind.

7. Verbindungen der Formel I, gemäss Anspruch 1, 2 und 4-6, in denen $R^6$ eine Gruppe $-CH_2R^{10}$ oder $-C(O)R^{10}$ ist.

8. Verbindungen der Formel I gemäss einem der Ansprüche 1, 2 und 4-7, in denen $R^6$ eine Gruppe $-C(O)R^{10}$, insbesondere Carbamoyl, ist.

9. Verbindungen der Formel II-1 gemäss den Ansprüchen 3-6, in denen $R^6$ eine Gruppe $-CH_2R^{10}$ $-C(O)R^{10}$ oder $-C(R^7)=C(R^8)COOR^9$ ist.

10. Verbindungen der Formel II-1 gemäss den Ansprüchen 3-6 und 9 in denen $R^6$ eine Gruppe $-C(O)R^{10}$ oder $-C(R^7)=C(R^8)COOR^9$ ist, worin $R^7$ und $R^9$ nieder-Alkyl und $R^8$ Wasserstoff sind, insbesondere Carbamoyl oder 2-Aethoxy-carbonyl-1-methylvinyl.

11. p-[(S)-3-[(S)-2-Phenyl-1-aziridinyl]butyl]benzamid und die physiologisch verträglichen Salze davon.

12. Verbindungen der Formel I und II-1 und physiologisch verträgliche Salze davon gemäß Anspruch 3, als Mittel zur Behandlung der Fettsucht und des Diabetes mellitus.

13. Verfahren zur Herstellung von Verbindungen der Formel I, dadurch gekennzeichnet, dass man eine Verbindung der Formel

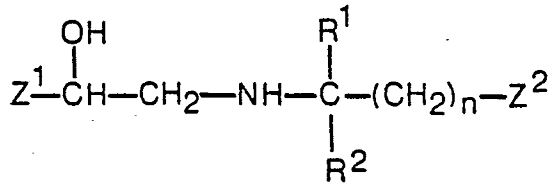 II

worin $Z^1$, $Z^2$, $R^1$, $R^2$ und n die in Anspruch 1 angegebene Bedeutung haben, dehydratisiert, gewünschtenfalls einen im Rest $Z^2$ enthaltene reaktionsfähigen Substituenten funktionell abwandelt und/oder das Reaktionsprodukt in ein physiologisch verträgliches Salz überführt.

14. Verfahren zur Herstellung von Verbindungen der Formel II-1 gemäß Anspruch 3, dadurch gekennzeichnet, dass man

a) eine Verbindung der Formel

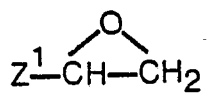 III

mit einer Verbindung der Formel

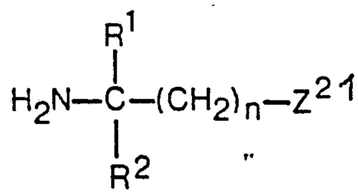 IV

umsetzt; oder

b) eine Verbindung einer der Formeln

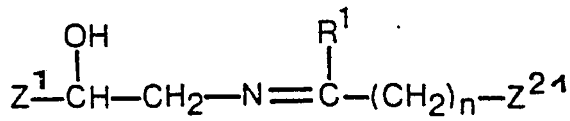 V

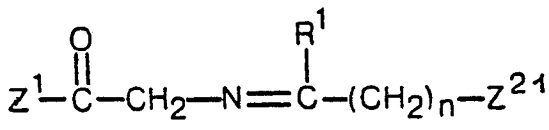

VI

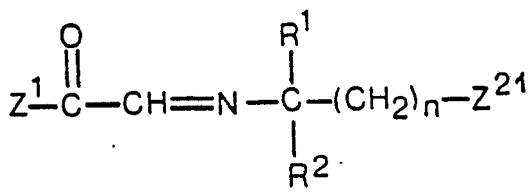

VII

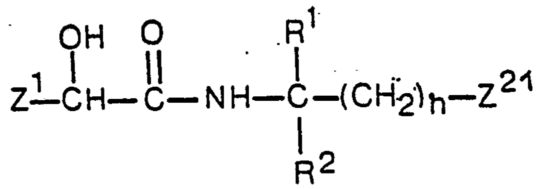

VIII

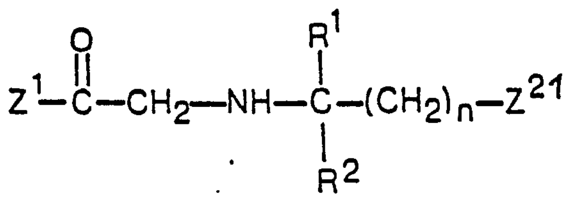

IX

reduziert,
wobei in den vorstehenden Formeln die Reste $Z^1$, $Z^{21}$ $R^1$, $R^2$ und n die im Anspruch 3 angegebene Bedeutung haben.

15. Pharmazeutische Präparate, g kennzeich net durch einen Gehalt an einer Verbindung der Formel I oder II-1 nach Anspruch 1 bzw. 3 oder an einem physiologisch verträglichen Salz davon.

**Patentansprüche** für den Vertragsstaat AT

1. Verfahren zur Herstellung von Verbindungen der Formel

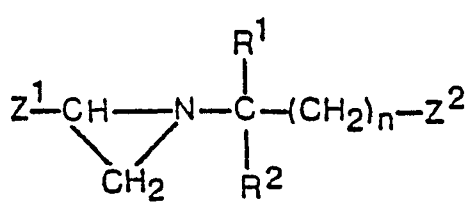

I

worin $Z^1$ ein Rest der Formel

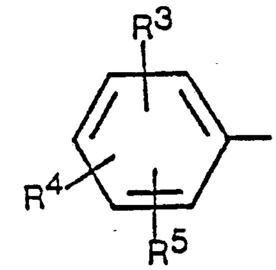

$Z^2$ ein Rest der Formel

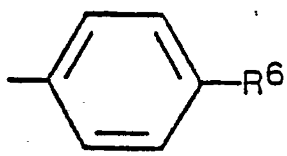 oder 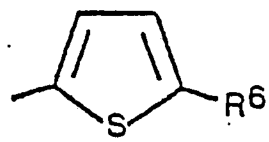

$R^1$ und $R^2$ Wasserstoff oder nieder-Alkyl; $R^3$ Wasserstoff, Halogen, Hydroxy, nieder-Alkyl, nieder-Alkoxy, Amino, nieder-Alkoxybenzylamino, Nitro oder Trifluormethyl; $R^4$ und $R^5$ Wasserstoff oder Halogen; $R^6$ nieder-Alkyl, nieder-Alkanoyl, Cyano, Hydroxy, oder einen Rest $-C(R^7)=C(R^8)COOR$ , $-CH_2R^{10}$ oder $-C(O)R^{10}$; $R^7$, $R^8$ und $R^9$ Wasserstoff oder nieder-Alkyl; $R^{10}$ Amino, Mono- oder Di-nieder-alkylamino, den Aetherrest eines niederaliphatischen Alkohols, wobei "nieder" sich auf Reste mit bis zu 6 C-Atome bezieht; und
n eine ganze Zahl von 1-4 ist, und von physiologisch verträglichen Salzen davon, dadurch gekennzeichnet, dass man eine Verbindung der Formel

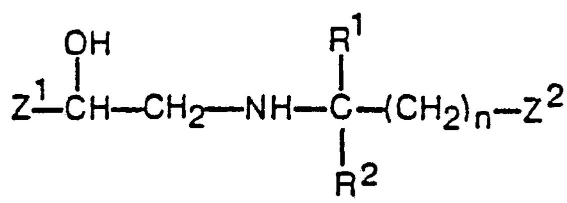

II

worin $Z^1$, $Z^2$, $R^1$, $R^2$ und n die oben angegebene Bedeutung haben,
dehydratisiert, gewünschtenfalls einen im Rest $Z^2$ enthaltene reaktionsfähigen Substituenten funktionell abwandelt und/oder das Reaktionsprodukt in ein physiologisch verträgliches Salz überführt.

2. Verfahren zur Herstellung der Verbindungen der Formel I nach Anspruch 1, in denen $R^1$ und $R^2$ Wasserstoff oder nieder-Alkyl, $R^3$ Wasserstoff, Halogen, Hydroxy, nieder-Alkyl, nieder-Alkoxy, Amino, nieder-Alkoxybenzylamino, Nitro oder Trifluormethyl, $R^4$ und $R^5$ Wasserstoff oder Halogen, $R^6$ Hydroxy, einen Rest $-C(R^7)=C(R^8)-COOR^9$, $-CH_2R^{10}$ oder $-C(O)R^{10}$ sind, und $R^7$, $R^8$, $R^9$ und $R^{10}$ die in Anspruch 1 angegebene Bedeutung haben, sowie der physiologisch verträglichen Salze davon.

3. Verfahren zur Herstellung von Verbindungen der Formel

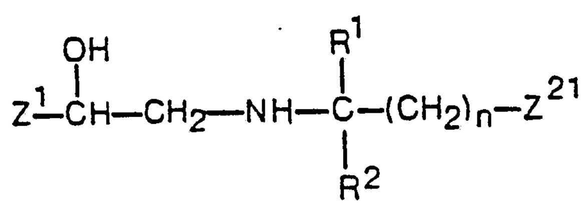

II—I

worin $Z^{21}$ einen Rest

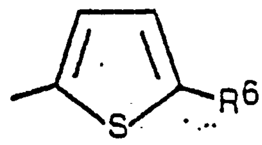

darstellt,
und $Z^1$, $R^1$, $R^2$, $R^6$ und $R^{10}$ die in Anspruch 1 angegebene Bedeutung haben,
und von physiologisch Verträglichen Salzen davon, dadurch gekennzeichnet, dass man
a) eine- Verbindung der Formel

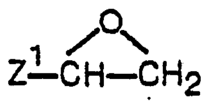 III

mit einer Verbindung der Formel

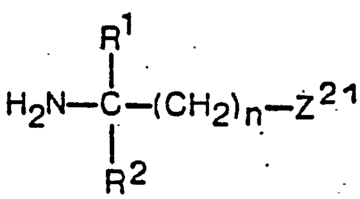 IV

umsetzt; oder
b) eine Verbindung einer der Formeln

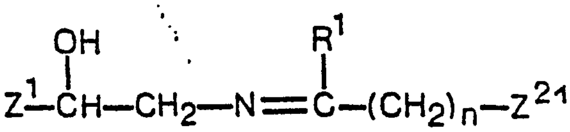 V

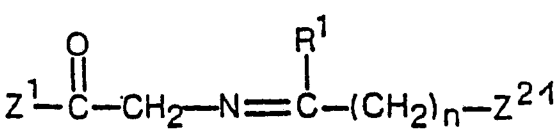 VI

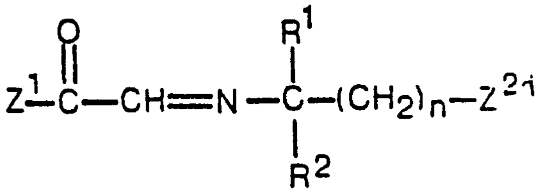 VII

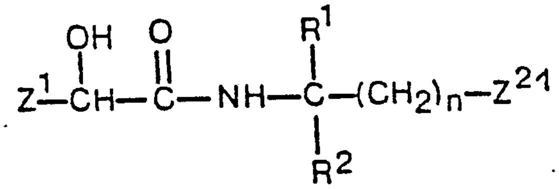

VIII

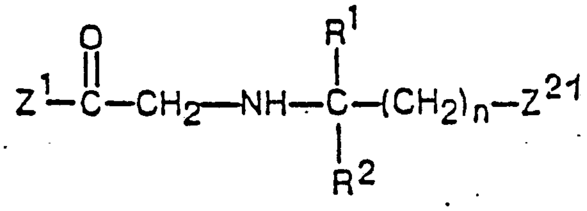

IX

reduziert,
wobei in den vorstehenden Formeln die Reste $Z^1$, $Z^{21}$, $R^1$, $R^2$ und n die früher angegebene Bedeutung haben.

4. Verfahren zur Herstellung von Verbindungen gemäss Anspruch 1, 2 oder 3 in denen $R^1$ Wasserstoff und $R^2$ Wasserstoff oder Methyl, insbesondere Methyl, sind.

5. Verfahren zur Herstellung von Verbindungen gemäss den Ansprüchen 1, 3 oder 4, in denen $R^3$ Wasserstoff, nieder-Alkyl, nieder-Alkoxy, Halogen oder Trifluormethyl, $R^4$ Wasserstoff oder Halogen, und $R^5$ Wasserstoff sind.

6. Verfahren zur Herstellung von Verbindungen gemäss einem der Ansprüche 1-5, in denen R Wasserstoff, Chlor oder Trifluormethyl, insbesondere in meta-Stellung, und $R^4$ und $R^5$ Wasserstoff sind.

7. Verfahren zur Herstellung von Verbindungen der Formel I, gemäss Anspruch 1, 2 und 4-6, in denen $R^6$ eine Gruppe $-CH_2R^{10}$ oder $-C(O)R^{10}$ ist.

8. Verfahren zur Herstellung von Verbindungen der Formel 1 gemäss einem der Ansprüche 1, 2 und 4-7, in denen $R^6$ eine Gruppe $-C(O)R^{10}$, insbesondere Carbamoyl, ist.

9. Verfahren zur Herstellung von Verbindungen der Formel II-1 gemäss den Ansprüchen 3-6, in denen $R^6$ eine Gruppe $-CH_2R^{10}$ $-C(O)R^{10}$ oder $-C(R^7)=C(R^8)COOR^9$ ist.

10. Verfahren zur Herstellung von Verbindungen der Formel II-1 gemäss den Ansprüchen 3-6 und 9, in denen $R^6$ eine Gruppe $-C(O)R^{10}$ oder $-C(R^7)=C(R^8)COOR^9$, ist, worin $R^7$ und $R^9$ nieder-Alkyl und $R^8$ Wasserstoff sind, insbesondere Carbamoyl oder 2-Aethoxycarbonyl-1-methylvinyl.

11. Verfahren nach Anspruch 1 zur Herstellung des p-((S)-3-[(S)-2-phenyl-1-aziridinyl]butyl]benzamids und der physiologisch verträglichen Salze davon, dadurch gekennzeichnet daß man ein entsprechendes Ausgangsmaterial der Formel II einsezt.

**Claims**

for the Contracting States BE CH DE FR GB IT LI LU NL SE

1. Compounds of the formula

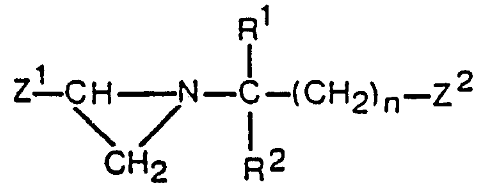

I

wherein $Z^1$ is a residue of the formula

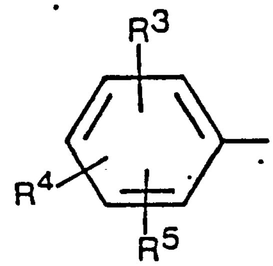

$Z^2$ is a residue of the formula

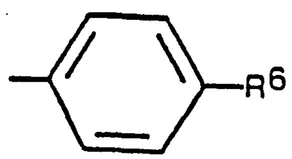

or

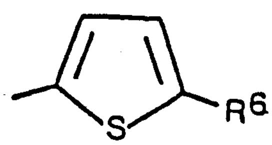

$R^1$ and $R^2$ are hydrogen or lower-alkyl; $R^3$ is hydrogen, halogen, hydroxy, lower-alkyl, lower-alkoxy, amino, lower-alkoxybenzylamino, nitro or trifluoromethyl. $R^4$ and $R^5$ are hydrogen or halogen; $R^6$ is lower-alkyl, lower-alkanoyl, cyano, hydroxy, or a residue $-C(R^7)=C(R^8)COOR^9$, $-CH_2R^{10}$ or $-C(O)R^{10}$; $R^7$, $R^8$ and $R^9$ are hydrogen or lower-alkyl; $R^{10}$ is amino. mono- or di-lower-alkylamino, the ether residue of a lower aliphatic alcohol, whereby "lower" denotes residues with up to 6 C-atoms; and n is a whole number of 1-4,

as well as physiologically compatible salts thereof.

2. Compounds of formula I according to claim 1, in which $R^1$ and $R^2$ are hydrogen or lower-alkyl, hydrogen, halogen, hydroxy, lower-alkyl, lower-alkoxy, amino, lower-alkoxybenzylamino, nitro or trifluoromethyl, $R^4$ and $R^5$ are hydrogen or halogen, $R^6$ is hydroxy, a residue $-C(R^7)=C(R^8)COOR^9$, $-CH_2R^{10}$ or $-C(O)R^{10}$, and $R^7$, $R^8$, $R^9$ and $R^{10}$ have the significance given in claim 1, as well as physiologically compatible salts thereof.

3. Compounds of the formula

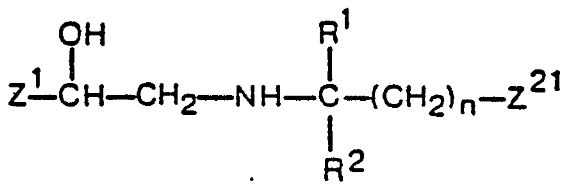

II-1

wherein $Z^{21}$ represents a residue

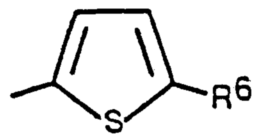

and $Z^1$, $R^1$, $R^2$, $R^6$ and $R^{10}$ have the significance given in claim 1, and their physiologically compatible salts.

4. Compounds in accordance with claim 1, 2 or 3, in which $R^1$ is hydrogen and $R^2$ is hydrogen or methyl, especially methyl.

5. Compounds in accordance with claims 1, 3 or 4, in which $R^3$ is hydrogen, lower-alkyl, lower-alkoxy, halogen or trifluoromethyl, $R^4$ is hydrogen or halogen, and $R^5$ is hydrogen.

6. Compounds in accordance with any one of claims 1-5, in which $R^3$ is hydrogen, chlorine or trifluoromethyl, especially in the meta-position, and $R^4$ and $R^5$ are hydrogen.

7. Compounds of formula I in accordance with claim 1, 2 and 4-6, in which $R^6$ is a group $-CR_2R^{10}$ or $-C(O)R^{10}$.

8. Compounds of formula I in accordance with any one of claims 1, 2 and 4-7, in which $R^6$ is a group $-C(O)R^{10}$, especially carbamoyl.

9. Compounds of formula II-1 in accordance with claims 3-6, in which $R^6$ is a group $-CR_2R^{10}$, $-C(O)R^{10}$ or $-C(R^7)=C(R^8)COOR^9$.

10. Compounds of formula II-1 in accordance with claims 3-6 and 9, in which $R^6$ is a group $-C(O)R^{10}$ or $-C(R^7)=C(R^8)COOR$ , wherein $R^7$ and $R^9$ are lower-alkyl and $R^8$ is hydrogen, especially carbamoyl or 2-ethoxycarbonyl-1-methylvinyl.

11. p-[(5)-3-[(S)-2-Phenyl-1-aziridinyl]butyl]-benzamide and the physiologically compatible salts thereof.

12. Compounds of formula I and II-1 and physiologically compatible salts thereof according to claim 1 or 3 as agents for the treatment of obesity and of diabetes mellitus.

13. A process for the manufacture of compounds of formula I, characterized by dehydrating a compound of the formula

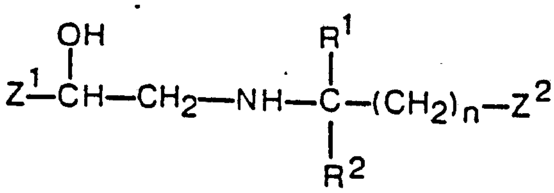

II

wherein $Z^1$, $Z^2$, $R^1$, $R^2$ and n have the significance given in claim 1, if desired, functionally modifying a reactive substituent present in the residue $Z^2$ and/or converting the reaction product into a physiologically compatible salt.

14. A process for the manufacture of compounds of formula II-1 in accordance with claim 3, characterized by

a) reacting a compound of the formula

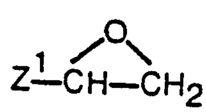

III

with a compound of the formula

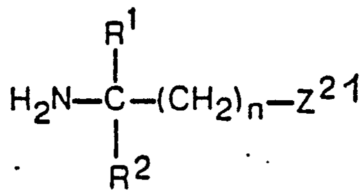

IV;

or

b) reducing a compound of one of the formulae

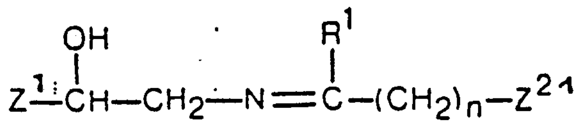

V

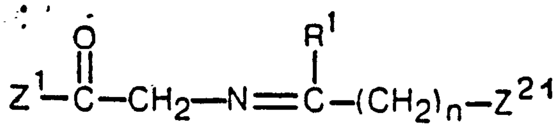

VI

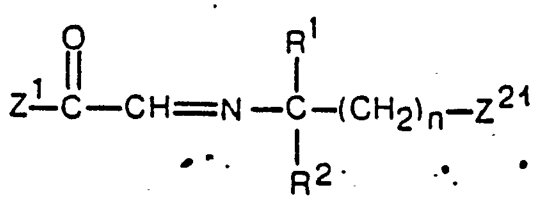

VII

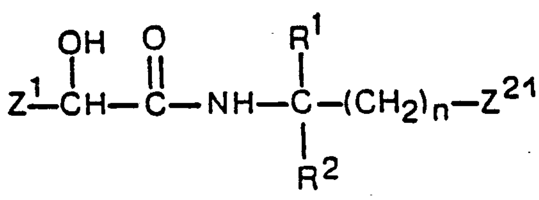

VIII

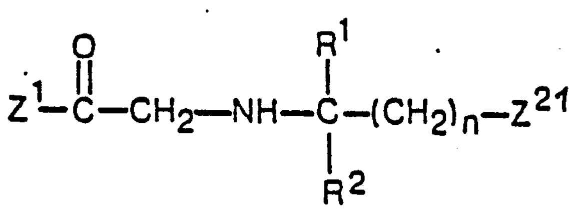

IX,

whereby in the foregoing formulae the residues $Z^1$, $Z^{21}$, $R^1$, $R^2$ and n have the significance given in claim 3.

15. Pharmaceutical preparations, characterized by a content of a compound of formula I or II-1 according to claim 1 or 3, respectively, or of a physiologically compatible salt thereof.

**Claims** for the Contracting State AT

1. A process for the manufacture of compounds of the formula

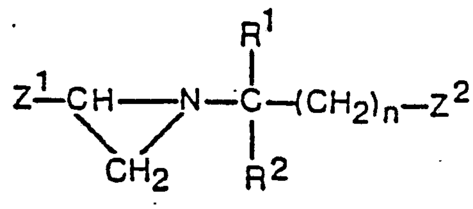

I

wherein $Z^1$ is a residue of the formula

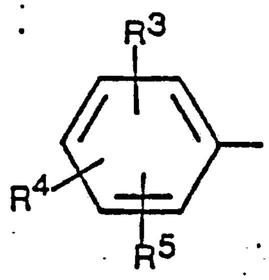

$Z^2$ is a residue of the formula

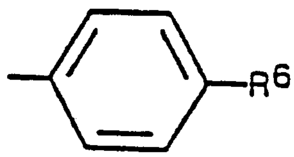

or

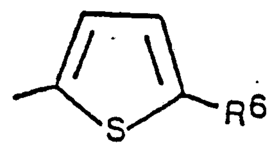

$R^1$ and $R^2$ are hydrogen or lower-alkyl; $R^3$ is hydrogen, halogen, hydroxy, lower-alkyl, lower-alkoxy amino, lower alkoxybenzylamino, nitro or trifluoromethyl; $R^4$ and $R^5$ are hydrogen or halogen; $R^6$ is lower-alkyl, lower-alkanoyl, cyano, hydroxy, or a residue $-C(R^7)=C(R^8)COOR^9$, $-CH_2R^{10}$ $-C(O)R^{10}$; $R^7$, $R^8$ and $R^9$ are hydrogen or lower-alkyl; $R^{10}$ is amino, mono- or di-lower-alkylamino, the ether residue of a lower aliphatic alcohol, whereby "lower" denotes residues with up to 6 C-atoms; and n is a whole number of 1-4,
and of physiologically compatible salts thereof, characterized by dehydrating a compound of the formula

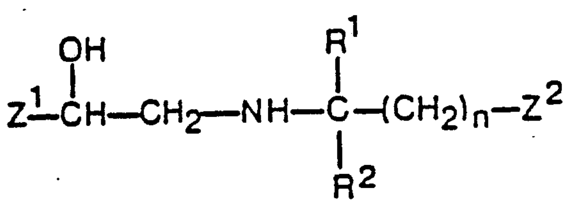

II

wherein $Z^1$, $Z^2$, $R^1$, $R^2$ and n have the significance given above,
if desired, functionally modifying a reactive substituent present in the residue $Z^2$ and/or converting the reaction product into a physiologically compatible salt.

2. A process for the manufacture of the compounds of formula I according to claim 1 in which $R^1$ and $R^2$ are hydrogen or lower-alkyl, $R^3$ is hydrogen, halogen, hydroxy, lower-alkyl, lower-alkoxy, amino, lower-alkoxybenzylamino, nitro or trifluoromethyl, $R^4$ and $R^5$ are hydrogen or halogen, $R^6$ is hydroxy, a residue $-C(R^7)=L(R^8)-(COOR^9$ $-CH_2R^{10}$ or $-C(O)R^{10}$, and $R^7$, $R^8$, $R^9$ and $R^{10}$ have the significance given in claim 1, as well as of the physiologically compatible salts thereof.

3. A process for the manufacture of compounds of the formula

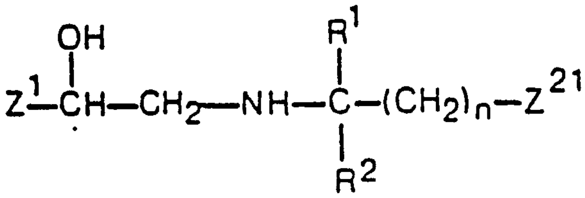

II-1

wherein $Z^{21}$ represents a residue

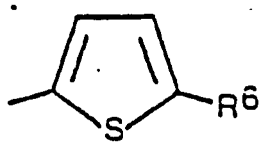

and $Z^1$, $R^1$, $R^2$, $R^6$ and $R^{10}$ have the significance given in claim 1, and of physiologically compatible salts thereof, characterized by
a) reacting a compound of the formula

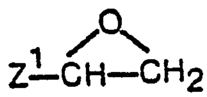

III

with a compound of the formula

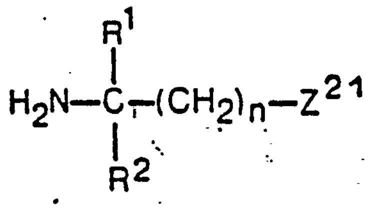

IV;

or
b) reducing a compound of one of the formulae

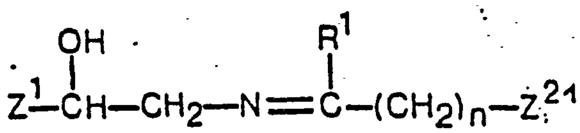

V

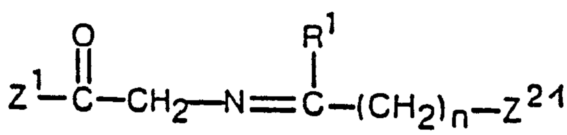

VI

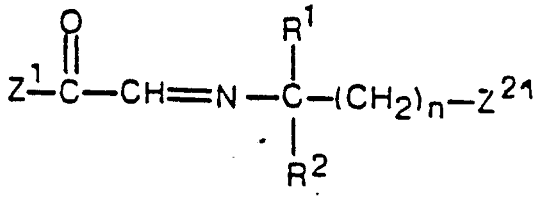 VII

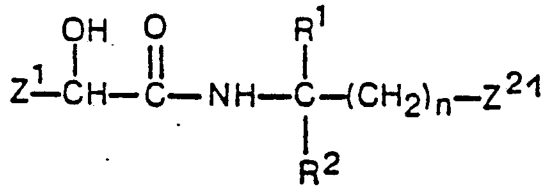 VIII

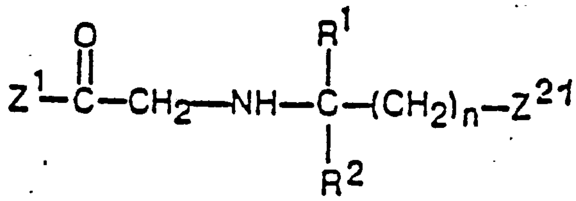 IX.

whereby in the foregoing formulae the residues $Z^1$, $Z^{21}$, $R^1$, $R^2$ and n have the significance given earlier.

4. A process for the manufacture of compounds in accordance with claim 1, 2 or 3, in which $R^1$ is hydrogen and $R^2$ is hydrogen or methyl, especially methyl.

5. A process for the manufacture of compounds in accordance with claims 1, 3 or 4, in which $R^3$ is hydrogen, lower-alkyl, lower-alkoxy, halogen or trifluoromethyl, $R^4$ is hydrogen or halogen, and $R^5$ is hydrogen.

6. A process for the manufacture of compounds in accordance with any one of claims 1-5, in which $R^3$ is hydrogen, chlorine or trifluoromethyl, especially in the meta-position, and $R^4$ and $R^5$ are hydrogen.

7. A process for the manufacture of compounds of formula I in accordance with claim 1, 2 and 4-6, in which $R^6$ is a group $-CR_2R^{10}$ or $-C(O)R^{10}$.

8. A process for the manufacture of compounds of formula I in accordance with any one of claims 1, 2 and 4-7, in which $R^6$ is a group $-C(O)R^{10}$, especially carbamoyl.

9. A process for the manufacture of compounds of formula II-I in accordance with claims 3-6, in which $R^6$ is a group $-CR_2R^{10}$, $-C(O)R^{10}$ or $-C(R^7)=C(R^8)COOR^9$.

10. A process for the manufacture of compounds of formula II-1 in accordance with claims 3-6 and 9, in which $R^6$ is a group $-C(O)R^{10}$ or $-C(R^7)=C(R^8)COOR^9$, wherein $R^7$ and $R^9$ are lower-alkyl and $R^8$ is hydrogen, especially carbamoyl or 2-ethoxycarbonyl-1-methylvinyl.

11. A process according to claim 1 for the manufacture of p-[(S)-3-[(S)-2-phenyl-1-aziridinyl]butyl]-benzamide and of the physiologically compatible salts thereof, characterized by using the corresponding starting material of formula II.

**Revendications**

pour les Etats contractants BE CH DE FR GB IT LI LU NL SE

1 - Composés de formule

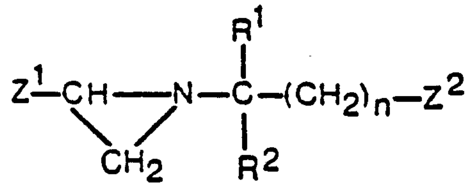

I

dans laquelle $Z^1$ représente un groupe de formule

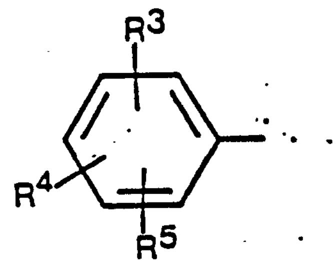

$Z^2$ représente un groupe de formule

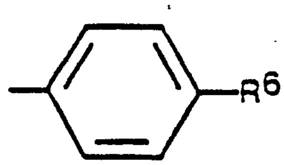

ou

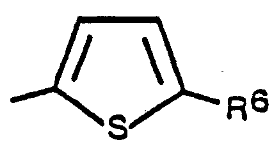

$R^1$ et $R^2$ représentent l'hydrogène ou un alkyle inférieur; $R^3$ représente l'hydrogène, un halogène, un groupe hydroxy, alkyle inférieur, alcoxy inférieur, amino, (alcoxy inférieur)-benzylamino, nitro ou trifluorométhyle; $R^4$ et $R^5$ représentent l'hydrogéne ou un halogène; $R^6$ représente un groupe alkyle inférieur, alcanoyle inférieur, cyano, hydroxy ou un groupe $-C(R^7)=C(R^8)COOR^9$,$-CH_2R^{10}$ ou $-C(O)R^{10}$; $R^7$, $R^8$ et $R^9$ représentent l'hydrogène ou un alkyle inférieur; $R^{10}$ représente un groupe amino, mono- ou di-(alkyle inférieur)-amino, le reste éther d'un alcool aliphatique inférieur, l'expression "inférieur" s'appliquant à un reste qui contient jusqu' à 6 atomes de carbone; et n est un nombre entier de 1 à 4, et leurs sels acceptables pour l'usage pharmaceutique.

2 - Composés de formule I selon la revendication 1, dans lesquels $R^1$ et $R^2$ représentent l'hydrogène ou un alkyle inférieur, $R^3$ représente l'hydrogène, un halogène, un groupe hydroxy, alkyle inférieur, alcoxy inférieur, amino, (alcoxy inférieur)-benzylamino, nitro ou trifluorométhyle, $R^4$ et $R^5$ représentent l'hydrogène ou un halogène, $R^6$ représente un groupe hydroxy, un groupe $-C(R^7)=C(R^8)COOR^9$, $-CH_2R^{10}$ ou $-C(O)R^{10}$, et $R^7$, $R^8$, $R^9$ et $R^{10}$ ont les significations indiquées dans la revendication 1, et leurs sels acceptables pour l'usage pharmaceutique.

3 - Composés de formule

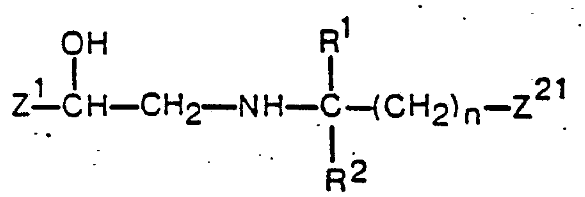

II-1

dans laquelle $Z^{21}$ représente un groupe

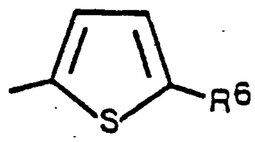

et $Z^1$, $R^1$, $R^2$, $R^6$ et $R^{10}$ ont les significations indiquées dans la revendication 1, et leurs sels acceptables pour l'usage pharmaceutique.

4 - Composés selon la revendication 1, 2 ou 3, dans lesquels $R^1$ représente l'hydrogène et $R^2$ l'hydrogène ou un groupe méthyle, plus spécialement un groupe méthyle.

5 - Composés selon les revendications 1, 3 ou 4, dans lesquels $R^3$ représente l'hydrogène, un alkyle inférieur, alcoxy inférieur, un halogène ou un groupe trifluorométhyle, $R^4$ représente l'hydrogène ou un halogène et $R^5$ l'hydrogène.

6 - Composés selon l'une des revendications 1 à 5, dans lesquels $R^3$ représente l'hydrogène, le chlore ou un trifluorométhyle, en particulier en position méta, et $R^4$ et $R^5$ représentent des atomes d'hydrogène.

7 - Composés de formule 1 selon la revendication 1, 2 et 4 à 6, dans lesquels $R^6$ représente un groupe $-CH_2R^{10}$ ou $-C(O)R^{10}$.

8 - Composés de formule 1 selon l'une des revendications 1, 2 et 4 à 7, dans lesquels $R^6$ représente un groupe $-C(O)R^{10}$, en particulier un groupe carbamoyle.

9 - Composés de formule II-1 selon les revendications 3 à 6, dans lesquels $R^6$ représente un groupe $-CH_2R^{10}$, $-C(O)R^{10}$ ou $-C(R^7){=}C(R^8)COOR^9$.

10 - Composés de formule II-1 selon les revendications 3 à 6 et 9, dans lesquels $R^6$ représente un groupe $-C(O)R^{10}$ ou $-C(R^7){=}C(R^8)COOR^9$, dans lesquels $R^7$ et $R^9$ représentent des groupes alkyle inférieurs et $R^8$ l'hydrogène, en particulier un groupe carbamoyle ou 2-éthoxycar-bonyl-1 -méthylvinyle.

11 - Le p-[(S)-3-[(S)-2-phényl-1-aziridinyl]-butyl]-benzamide et ses sels acceptables pour l'usage pharmaceutique.

12 - Composés de formules I et II-1 et leurs sels acceptables pour l'usage pharmaceutique selon la revendication 1 ou 3, en tant qu'agents pour le traitement de l'obésité et du diabète sucré.

13 - Procédé de préparation des composés de formule I, caractérisé en ce que l'on déshydrate un composé de formule

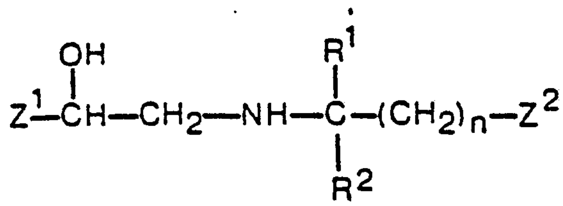

II

dans laquelle $Z^1$, $Z^2$, $R^1$, $R^2$ et n ont les significations indiquées dans la revendication 1,

et si on le désire, on soumet un substituant réactif contenu dans le reste $Z^2$ à une modification fonctionnelle et/ou on convertit le produit de réaction en un sel acceptable pour l'usage pharmaceutique.

14 - Procédé de préparation des composés de formule II-1 selon la revendication 3, caractérisé en ce que

a) on fait réagir un composé de formule

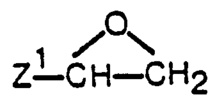

III

avec un composé de formule

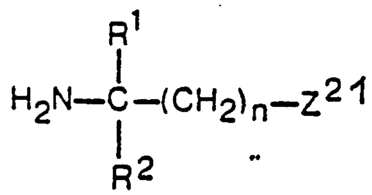

IV

; ou

b) on réduit un composé répondant à l'une des formules

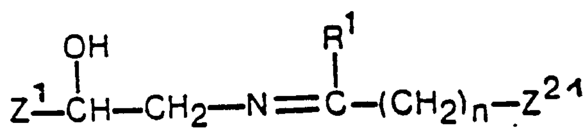

V

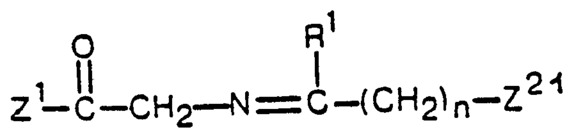

VI

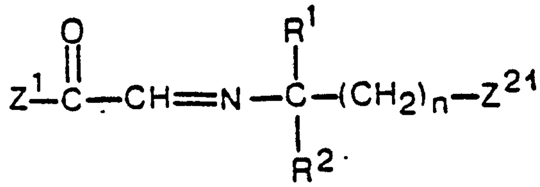

VII

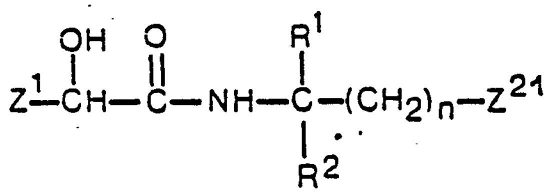

VIII

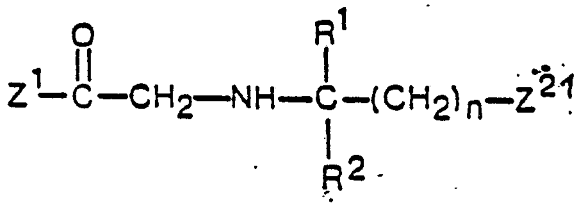

IX

les symboles $Z^1$, $Z^{21}$, $R^1$, $R^2$ et n ayant dans les formules ci-dessus les significations indiquées dans la revendication 3.

15 - Compositions pharmaceutiques caractérisées en ce qu' elles contiennent un composé de formule I ou II-1 selon les revendications respectives 1 et 3 ou un sel acceptable pour l'usage pharmaceutique d'un tel composé.

**Revendications**: Pour l'Etat contractant AT

1 - Procédé de préparation de composés de formule

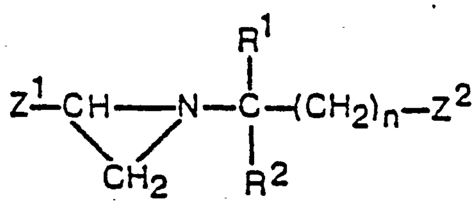

I

dans laquelle $Z^1$ représente un groupe de formule

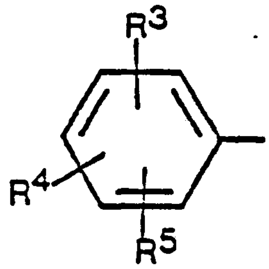

$Z^2$ un reste de formule

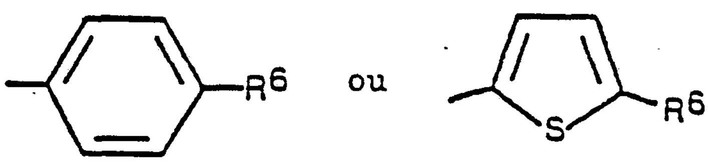

$R^1$ et $R^2$ représentent l'hydrogène ou un alkyle inférieur; $R^3$ représente l'hydrogène, un halogène, un groupe hydroxy, alkyle inférieur, alcoxy inférieur, amino, (alcoxy inférieur)-benzylamino, nitro ou trifluorométhyle; $R^4$ et $R^5$ représentent l'hydrogène ou des halogènes; $R^6$ représente un alkyle inférieur, alcanoyle inférieur, cyano, hydroxy ou un groupe $-C(R^7)=C(R^8)COOR^9$, $-CH_2R^{10}$ ou $-C(O)R^{10}$; $R^7$, $R^8$ et $R^9$ représentent l'hydrogène ou des groupes alkyle inférieurs; $R^{10}$ représente un groupe amino, mono- ou di-(alkyle inférieur)-amino, le reste éther d'un alcool aliphatique inférieur, l'expression "inférieur" s'appliquant à des restes qui contiennent jusqu'à 6 atomes de carbone; et

n est un nombre entier de 1 à 4,

et de leurs sels acceptables pour l'usage pharmaceutique, caractérisé en ce que l'on déshydrate un composé de formule

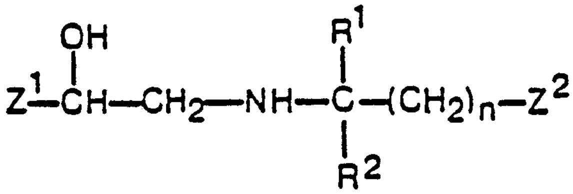

II

dans laquelle $Z^1$, $Z^2$, $R^1$, $R^2$ et n ont les significations indiquées ci-dessus, et si on le desire on soumet un substituant réactif contenu dans le reste $Z^2$ à une modification fonctionnelle et/ou on convertit le produit de réaction en un sel acceptable pour l'usage pharmaceutique.

2 - Procédé de préparation des composés de formule I selon la revendication 1, dans lesquels $R^1$ et $R^2$ représentent l'hydrogène ou un alkyle inférieur, $R^3$ représente l'hydrogène, un halogène, un groupe hydroxy, alkyle inférieur, alcoxy inférieur, amino, (alcoxy inférieur)-benzylamino, nitro ou trifluorométhyle, $R^4$ et $R^5$ représentent l'hydrogène ou un halogène, $R^6$ représente un groupe hydroxy, un groupe $-C(R^7)=C(R^8)COOR^9$, $-CH_2R^{10}$ ou $-C(O)R^{10}$ et $R^7$, $R^8$, $R^9$ et $R^{10}$ ont les significations indiquées dans la revendication 1, et de leurs sels acceptables pour l'usage pharmaceutique.

3 - Procédé de préparation des composés de formule

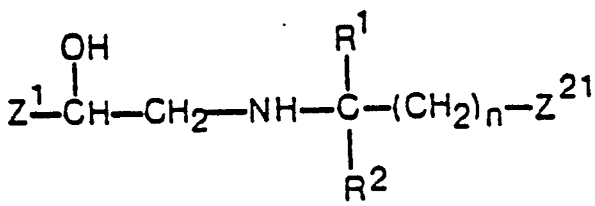

II—I

dans laquelle $Z^{21}$ représente un groupe

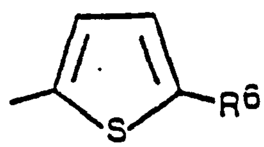

et $Z^1$, $R^1$, $R^2$, $R^6$ et $R^{10}$ ont les significations indiquées dans la revendication 1,
et de leurs sels acceptables pour l'usage pharmaceutique, caractérisé en ce que:
a) on fait réagir un composé de formule

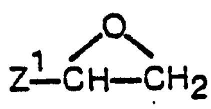

III.

avec un composé de formule

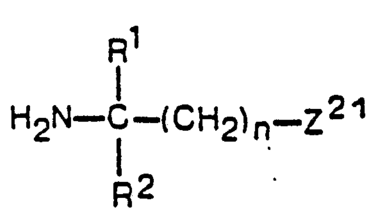

IV

ou bien
b) on réduit un composé répondant à l'une des formules

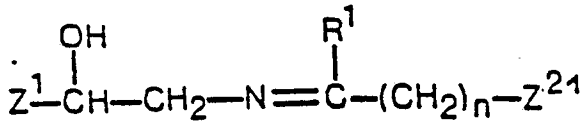

V

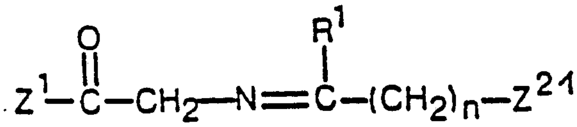

VI

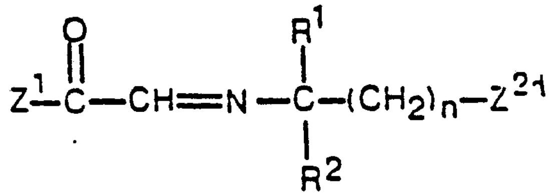

VII

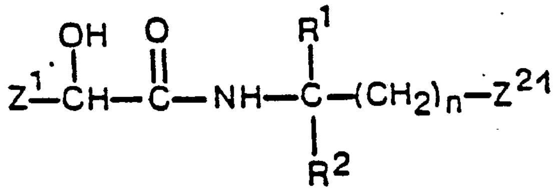

VIII

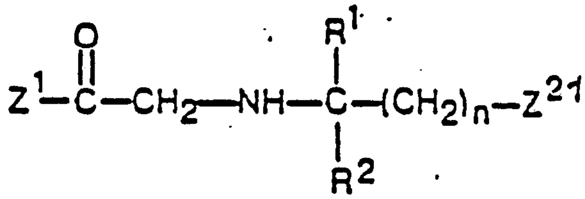

IX

les symboles $Z^1$, $Z^{21}$, $R^1$, $R^2$ et n ayant dans les formules ci-dessus les significations indiquées précédemment.

4 - Procédé de préparation des composés selon la revendication 1, 2 ou 3, dans lesquels $R^1$ représente l'hydrogène et $R^2$ l'hydrogène ou un groupe méthyle, plus spécialement un groupe méthyle.

5 - Procédé de préparation des composés selon les revendications 1, 3 ou 4, dans lesquels $R^3$ représente l'hydrogène, un groupe alkyle inférieur, alcoxy inférieur, un halogène ou un groupe trifluorométhyle, $R^4$ représente l'hydrogène ou un halogène, et $R^5$ l'hydrogène.

6 - Procédé de préparation des composés selon l'une des revendications 1 à 5, dans lesquels $R^3$ représente l'hydrogène, le chlore ou un groupe trifluorométhyle, en particulier en position méta, et $R^4$ et $R^5$ représentent l'hydrogène.

7 - Procédé de préparation des composés de formule I selon les revendications 1, 2 et 4 à 6, dans lesquels $R^6$ représente un groupe $-CH_2R^{10}$ ou $-C(O)R^{10}$.

8 - Procédé de préparation des composés de formule I selon l'une des revendications 1, 2 et 4 à 7, dans lesquels $R^6$ represente un groupe $-C(O)R^{10}$, en particulier carbamoyle.

9 - Procédé de préparation des composés de formule II-1 selon les revendications 3 à 6, dans lesquels $R^6$ représente un groupe $-CH_2R^{10}$, $-C(O)R^{10}$ ou $-C(R^7)-C(R^8)COOR^9$.

10 - Procédé de préparation des composés de formule II-1 selon les revendications 3 à 6 et 9, dans lesquels $R^6$ représente un groupe $-C(O)R^{10}$ ou $-C(R^7)= C(R^8)COOR^9$, dans lequel $R^7$ et $R^9$ représentent des groupes alkyle inférieurs et $R^8$ représente l'hydrogène, en particulier carbamoyle ou 2-éthoxycarbonyl-1-méthylvinyle.

11 - Procédé selon la revendication 1 pour préparer le p-[(S)-3-[(S)-2-phényl-1-aziridinyl]-butyl]-benzamide et ses sels acceptables pour l'usage pharmaceutique, caractérisè en ce que l'on met en oeuvre un composé de départ correspondant de formule II.